# EUROPEAN PATENT APPLICATION

(11) **EP 4 023 631 A1**
(43) Date of publication of application: **06.07.2022**
(21) Application number: 20872158.9
(22) Date of filing: 29.09.2020
(51) Int. Cl.: C07C 233/09, C07D 295/15, C07D 211/58, C07D 211/38, C07C 233/25, C07D 295/145, A61P 29/00, A61P 25/00, A61P 31/22, A61P 3/10, A61P 31/18, A61P 35/00, A61P 15/00, A61P 19/02, A61P 19/10, A61P 25/04, A61P 21/00, A61K 31/223, A61K 31/40, A61K 31/4545

(54) **TRPV1 AGONIST AND PREPARATION METHOD THEREFOR AND USE THEREOF**

(30) Priority: 30.09.2019 CN 201910944854
(71) Applicant: Nanjing Delova Biotech Co. Ltd., Nanjing, Jiangsu 210033 (CN)
(72) Inventor: WANG, Qingsong, Nanjing, Jiangsu 210033 (CN); ZOU, Limin, Nanjing, Jiangsu 210033 (CN); WU, Qu, Nanjing, Jiangsu 210033 (CN)
(74) Representative: Gille Hrabal Partnerschaftsgesellschaft mbB Patentanwälte
(86) International application number: PCT/CN2020/119064
(87) International publication number: WO 2021/063373

(57) **Abstract**

The present invention relates to a compound represented by formula I and a stereoisomer, tautomer, solvate, polymorph thereof or a pharmaceutically acceptable salt thereof, a pharmaceutical composition containing same, a preparation method therefor, and the medical use thereof, wherein the structure of formula I is as follows.

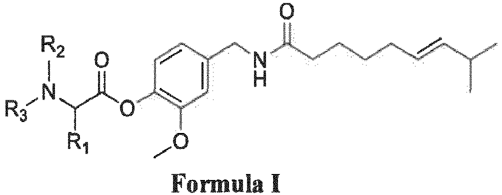

## Description

The present application claims priority to Chinese Patent Application No. 201910944854.8 filed with China National Intellectual Property Administration on Sep. 30, 2019 and entitled "TRPV1 AGONIST AND PREPARATION METHOD THEREFOR AND USE THEREOF", which is incorporated herein by reference in its entirety.

### TECHNICAL FIELD

The present invention relates to the field of pharmaceutical chemistry, and particularly relates to a TRPV1 agonist and a preparation method therefor and use thereof.

### BACKGROUND

Pain is the most common cause of medical attendance of patients. Pain is generally divided into acute pain (less than 1 month) and chronic pain. Acute pain is often accompanied by anxiety and sympathetic excitation (such as accelerated heartbeat and breathing, elevated blood pressure, sweating and dilated pupils). Chronic pain generally does not cause sympathetic excitation, but may be accompanied by autonomic manifestations (such as fatigue, hyposexuality, and anorexia) and depression. Pain tolerance can vary significantly from person to person. At present, the commonly used clinical treatment is the analgesic pump treatment. However, drugs contained in the analgesic pump are mainly opioid analgesics and some auxiliary analgesics such as tramadol, which are effective, but come with a series of side effects such as respiratory depression, nausea and vomiting, hypotension and potential addiction.

TRPV1 (transient receptor potential vanilloid 1) is a membrane channel receptor closely related to pain transmission, and is mainly distributed in nociceptive sensory neurons. The receptor plays an important role in mediating various pains such as inflammatory pain, visceral pain and cancer pain, and thus becomes one of the current hot spots in the research of pain mechanisms and the development of analgesic drugs.

A TRPV1 agonist of the following structure, chemically named *trans*-8-methyl-*N*-vanillyl-6-nonenamide, has been found:

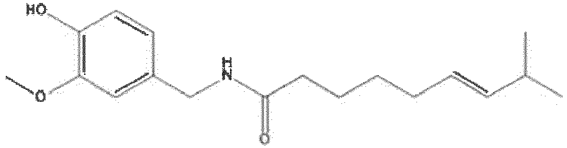

*Trans*-8-methyl-*N*-vanillyl-6-nonenamide has LogP of 3.8, and is highly lipophilic. Therefore, *trans*-8-methyl-*N*-vanillyl-6-nonenamide is practically insoluble in water, with the solubility in water of 0.00841 mg/mL at 25 °C, is soluble in ethanol, ether, benzene and chloroform, and is slightly soluble in carbon disulfide.

*Trans*-8-methyl-*N*-vanillyl-6-nonenamide can act on peripheral afferent nerve fibers that transmit chemical and thermal stimulations and signals from baroreceptors, activate TRPV1, and cause the opening of calcium channels, the influx of calcium ions, and the increase of cytosolic calcium concentration, thereby promoting the release of neuropeptides from neurons and their fibers, such as substance P, neurokinins, calcitonin gene-related peptide (CGRP), vasoactive intestinal peptide and excitatory amino acids (such as glutamic acid and aspartic acid), depleting and inhibiting their formation, and blocking the transmission pathway of pains from peripheral to central nerves. A certain dose of *trans*-8-methyl-*N*-vanillyl-6-nonenamide binds to TRPV1 to cause terminal desensitization of nociceptive neurons, thereby exerting an analgesic effect.

Although *trans*-8-methyl-*N*-vanillyl-6-nonenamide has a good analgesic effect, its further application is limited due to poor water solubility resulting in difficult absorption by oral administration and high irritation, and insufficient activity, low absorption, rapid metabolism and low bioavailability *in vivo* as demonstrated in *in vivo* studies. Therefore, there is a need to develop a *trans*-8-methyl-*N*-vanillyl-6-nonenamide derivative having good *in vivo* activity, bioavailability and druggability as a TRPV1 agonist.

### SUMMARY

In order to solve the problems in the prior art, the present invention provides a compound of formula I, or a stereoisomer, a tautomer, a solvate, a polymorph or a pharmaceutically acceptable salt thereof: 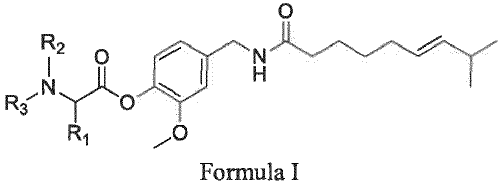 wherein,
R₁ is selected from H, halogen, hydroxyl, amino, and the following groups unsubstituted or optionally substituted with one, two or more R: a (C₁-C₁₂)aliphatic hydrocarbyl, a (C₁-C₁₂)aliphatic hydrocarbyl optionally comprising one, two or more heteroatoms, a C₃₋₁₂ cycloalkyl, a 3-12 membered heterocycloalkyl, a C₆₋₂₀ aryl, a 5-14 membered heteroaryl, S(=O)₂NH₂, -S(=O)₂NH-(C₁-C₁₂)aliphatic hydrocarbyl, and -S(=O)₂N((C₁-C₁₂)aliphatic hydrocarbyl)₂;
R₂ and R₃ are each independently selected from H, halogen, hydroxyl, amino, and the following groups unsubstituted or optionally substituted with one, two or more R: a (C₁-C₁₂)aliphatic hydrocarbyl, a (C₁-C₁₂)aliphatic hydrocarbyl optionally comprising one, two or more heteroatoms, a C₃₋₁₂ cycloalkyl, a 3-12 membered heterocycloalkyl, a C₆₋₂₀ aryl, a 5-14 membered heteroaryl, S(=O)₂NH₂, -S(=O)₂NH-(C₁-C₁₂)aliphatic hydrocarbyl, and -S(=O)₂N((C₁-C₁₂)aliphatic hydrocarbyl)₂, and at least one of R₂ and R₃ is not H; or R₂ and R₃, together with an N atom connected thereto, form an *N*-containing 3-12 membered heterocycloalkyl or an *N*-containing 5-14 membered heteroaryl unsubstituted or optionally substituted with one, two or more R;
The R is selected from halogen, CN, OH, NH₂, COOH, =O, a (C₁-C₁₂)aliphatic hydrocarbyl, a (C₁-C₁₂)aliphatic hydrocarbyl optionally comprising one, two or more heteroatoms, a C₃₋₁₂ cycloalkyl, a 3-12 membered heterocycloalkyl, a C₆₋₂₀ aryl, and a 5-14 membered heteroaryl.

According to an embodiment of the present invention, the *N*-containing 3-12 membered heterocycloalkyl or the *N*-containing 5-14 membered heteroaryl may be selected from an *N*-containing 5-6 membered heterocycloalkyl and an *N*-containing 5-6 membered heteroaryl, for example, tetrahydropyrrole, piperidine, morpholine, pyridine and pyrimidine.

According to an embodiment of the present invention, R₂ and R₃ are each independently selected from the following groups unsubstituted or optionally substituted with one, two or more R: a (C₁-C₁₂)aliphatic hydrocarbyl, and a (C₁-C₁₂)aliphatic hydrocarbyl optionally comprising one, two or more heteroatoms; for example, R₂ and R₃ are each independently selected from methyl, ethyl and propyl.

According to an embodiment of the present invention, the halogen is selected from F, Cl, Br and I. According to an embodiment of the present invention, the (C₁-C₁₂) aliphatic hydrocarbyl may be selected from a C₁-C₁₂ alkyl, a C₂-C₁₂ alkenyl and a C₂-C₁₂ alkynyl, and in some embodiments, may be selected from a (C₁-C₆)alkyl, a (C₂-C₆)alkenyl and a (C₂-C₆)alkynyl.

According to an embodiment of the present invention, the "(C₁-C₁₂)aliphatic hydrocarbyl optionally comprising one, two or more heteroatoms" may be selected from a (C₁-C₆)aliphatic hydrocarbyloxy, a (C₁-C₆)aliphatic hydrocarbylthio, a (C₁-C₆)aliphatic hydrocarbyloxy(C₁-C₆)aliphatic hydrocarbyl, a (C₁-C₆)aliphatic hydrocarbylthio(C₁-C₆)aliphatic hydrocarbyl, an *N*-(C₁-C₃)aliphatic hydrocarbylamino (C₁-C₆)aliphatic hydrocarbyl, an *N*,*N*-di-(C₁-C₃)aliphatic hydrocarbylamino(C₁-C₆)aliphatic hydrocarbyl, a (C₁-C₆)aliphatic hydrocarbyl-NH-, and an N((C₁-C₆)aliphatic hydrocarbyl)₂. In some embodiments, the (C₁-C₁₂)aliphatic hydrocarbyl substituted with one, two or more R and optionally comprising one, two or more heteroatoms may be selected from a -(C₁-C₆)aliphatic hydrocarbyl OC(=O)NH₂, a -(C₁-C₆)aliphatic hydrocarbyl OC(=O)NH(C₁-C₆)aliphatic hydrocarbyl, a -(C₁-C₆)aliphatic hydrocarbyl OC(=O)N((C₁-C₆)aliphatic hydrocarbyl)₂, a -(C₁-C₆)aliphatic hydrocarbyl OC(=O)(C₁-C₆) aliphatic hydrocarbyl, and a -(C₁-C₆)aliphatic hydrocarbyl C(=O)O(C₁-C₆)aliphatic hydrocarbyl.

According to an embodiment of the present invention, R₁ may be selected from methyl, ethyl, *n*-propyl, isopropyl, *n*-butyl, isobutyl, *tert*-butyl*, n*-pentyl, isopentyl, neopentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, phenyl, thienyl, furyl, pyrrolyl, pyridinyl, pyridazinyl, pyrimidinyl, pyrazinyl, and triazinyl.

According to an embodiment of the present invention, R₂ and R₃ may be each independently selected from the following groups unsubstituted or optionally substituted with one, two or more R: methyl, ethyl, *n*-propyl, isopropyl, *n*-butyl, isobutyl, *tert-butyl, n*-pentyl, isopentyl, neopentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, and 1-hexynyl; or R₂ and R₃, together with an N atom connected thereto, form the following groups optionally substituted with one, two or more R: tetrahydropyrrolyl, piperidinyl, and 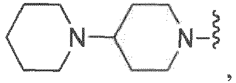 for example, may be selected from 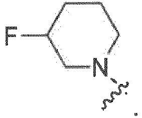 For the compound of formula I, or the stereoisomer, the tautomer, the solvate, the polymorph or the pharmaceutically acceptable salt thereof disclosed herein, the compound of formula I may be specifically selected from the following structures: 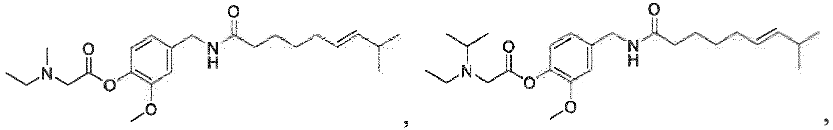 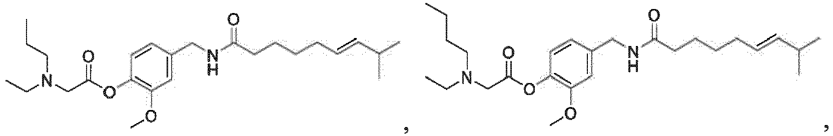 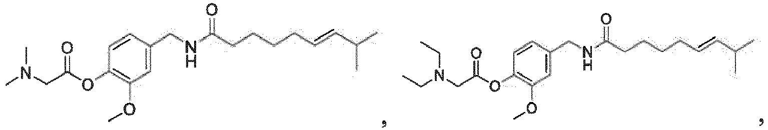 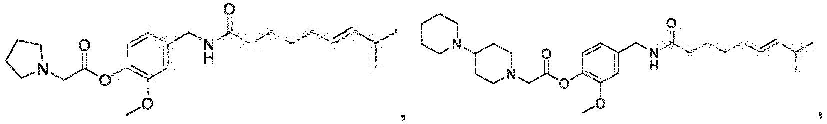 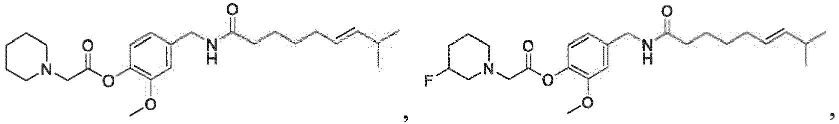 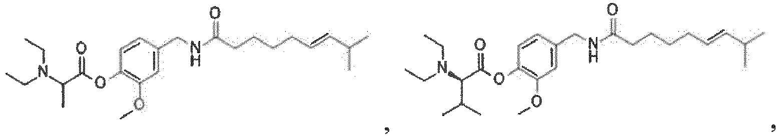 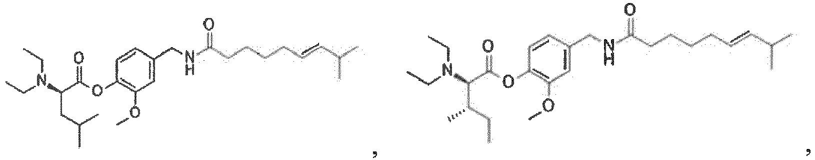 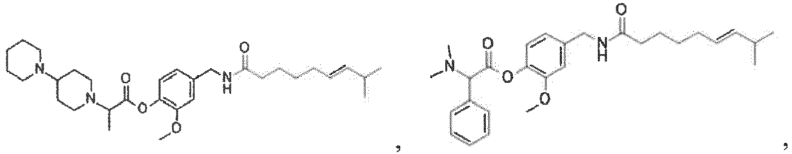 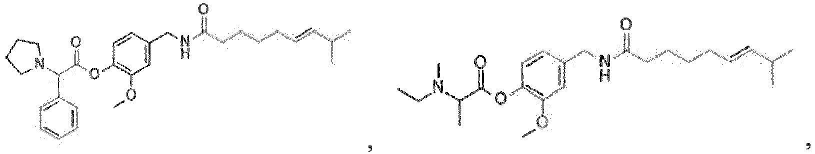 and

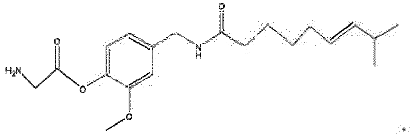

According to an embodiment of the present invention, the pharmaceutically acceptable salt of the compound of formula I may be selected from inorganic acid salts and organic acid salts, such as hydrochloride, oxalate, formate, and acetate. According to an embodiment of the present invention, the pharmaceutically acceptable salt of the compound of formula I may be further selected from the following structure:

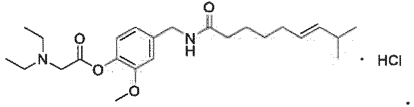

The present invention also provides a preparation method for the compound of formula I, or the stereoisomer, the tautomer, the solvate, the polymorph or the pharmaceutically acceptable salt thereof, but the preparation method is not limited to the method described below.

In some embodiments, the preparation method for the compound of formula I, or the stereoisomer, the tautomer, the solvate, the polymorph or the pharmaceutically acceptable salt thereof, comprises the following steps:
(1) reacting 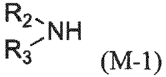 with chloroacetic acid to give 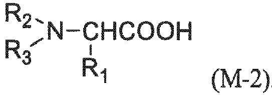 ; R₁, R₂ and R₃ are defined as those of the compound of formula I above;
(2) reacting the M-2 with *trans*-8-methyl-*N*-vanillyl-6-nonenamide to give a compound of formula I, or a stereoisomer, a tautomer, a polymorph, a solvate or a pharmaceutically acceptable salt thereof;
(3) optionally, reacting the compound of formula (I) with a solvent or a pharmaceutically acceptable salt to give a corresponding solvate or a corresponding pharmaceutically acceptable salt of the compound of formula (I).

According to an embodiment of the present invention, in the step (1), the reaction temperature may be room temperature, and the reaction time may be selected from 4-8 h, for example, 6 h; in the step (2), the reaction temperature may be room temperature, the reaction time may be selected from 2-4 h, for example, 3 h, and the reaction may be performed in the presence of EDCI, DMAP, and DCM. According to an embodiment of the present invention, the above preparation method may further comprise forming a salt of the compound of formula I with an acid or a base, for example, HCl gas may be purged to give a hydrochloride of the compound of formula I, and the reaction for forming the hydrochloride may be performed in the presence of sodium chloride and concentrated sulfuric acid, the reaction temperature is room temperature, and the reaction time is 0.5-2 h.

The present invention further provides a pharmaceutical composition comprising the compound of formula I, or the stereoisomer, the tautomer, the solvate, the polymorph or the pharmaceutically acceptable salt thereof disclosed herein.

In some embodiments, the pharmaceutical composition disclosed herein further comprises a therapeutically effective amount of the compound of formula I, or the stereoisomer, the tautomer, the solvate, the polymorph or the pharmaceutically acceptable salt thereof disclosed herein, and a pharmaceutically acceptable carrier.

The present invention further provides a use of the compound of formula I, or the stereoisomer, the tautomer, the solvate, the polymorph or the pharmaceutically acceptable salt thereof, or the pharmaceutical composition in preparing a medicament for treating diseases or conditions responsive to *trans*-8-methyl-*N*-vanillyl-6-nonenamide.

The present invention further provides a use of the compound of formula I, or the stereoisomer, the tautomer, the solvate, the polymorph or the pharmaceutically acceptable salt thereof, or the pharmaceutical composition in preparing a TRPV1 agonist. In some embodiments, the TRPV1 agonist may be used to modulate the activity of a transient receptor potential vanilloid 1 (TRPV1) receptor.

According to an embodiment of the present invention, the compound of formula I, or the stereoisomer, the tautomer, the solvate, the polymorph or the pharmaceutically acceptable salt thereof, or the pharmaceutical composition may be administered by the following routes: intravenous injection, subcutaneous injection, intramuscular injection, intraperitoneal injection, perineural injection, neuraxial injection, intra-articular injection, oral administration or topical administration.

The present invention also provides a method for treating diseases or conditions responsive to *trans*-8-methyl-*N*-vanillyl-6-nonenamide comprising administering to the subject a therapeutically effective amount of the compound of formula I, or the stereoisomer, the tautomer, the solvate, the polymorph or the pharmaceutically acceptable salt thereof.

The present invention also provides a method for treating diseases or conditions associated with the modulation of TRPV1 activity comprising administering to the subject a therapeutically effective amount of the compound of formula I, or the stereoisomer, the tautomer, the solvate, the polymorph or the pharmaceutically acceptable salt thereof.

According to an embodiment of the present invention, the disease or condition is selected from post-operative pain, neuropathic pain, post-herpetic neuralgia, diabetic neuropathy, HIV-associated neuropathy, complex regional pain syndrome, cancer, nerve injury, cancer chemotherapy, vulvodynia, trauma, surgery, chronic musculoskeletal pain, lower back pain, osteoarthritis and rheumatoid arthritis-related conditions.

According to an embodiment of the present invention, the method may comprise administering the compound disclosed alone or in combination with one or more other chemotherapeutic agents. Multiple drugs may be administered simultaneously or successively.

### Terminology

Unless otherwise stated, the definitions of groups and terms described in the specification and claims of the present application, including definitions thereof as examples, exemplary definitions, preferred definitions, definitions documented in tables, definitions of specific compounds in the examples, and the like, may be arbitrarily combined and incorporated with each other. The definitions of groups and the structures of the compounds in such combinations and incorporations should fall within the scope of the present specification.

The term "more" refers to three or more.

The term "halogen" refers to F, Cl, Br and I. In other words, F, Cl, Br and I may be described as "halogen" in the specification.

The term "aliphatic hydrocarbyl" includes saturated or unsaturated, and linear or branched or cyclic hydrocarbyl groups. The aliphatic hydrocarbyl may be selected from alkyl, alkenyl, alkynyl, and the like, has preferably 1-12 or 1-10 carbon atoms, and more preferably 1-6 carbon atoms, and specifically may include but is not limited to the following groups: methyl, ethyl, n-propyl, isopropyl, *n*-butyl, isobutyl, *tert-butyl,* n-pentyl, isopentyl, neopentyl, *n*-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, cyclopropyl, cyclobutyl, cyclopentyl and cyclohexyl;

The "aliphatic hydrocarbyl" may optionally comprise one, two or more heteroatoms (which may be construed as optional insertion of heteroatoms into any C-C bond and C-H bond of the aliphatic hydrocarbyl). Suitable heteroatoms will be apparent to those skilled in the art and include, for example, sulfur, nitrogen, oxygen, phosphorus and silicon. The aliphatic hydrocarbyl comprising heteroatoms may be selected from the following groups: a (C₁-C₆)aliphatic hydrocarbyloxy, a (C₁-C₆)aliphatic hydrocarbylthio, a (C₁-C₆)aliphatic hydrocarbyloxy(C₁-C₆)aliphatic hydrocarbyl, a (C₁-C₆)aliphatic hydrocarbylthio(C₁-C₆)aliphatic hydrocarbyl, an *N*-(C₁-C₃)aliphatic hydrocarbylamino(C₁-C₆)aliphatic hydrocarbyl, and an *N*,*N*-di-(C₁-C₃)aliphatic hydrocarbylamino(C₁-C₆)aliphatic hydrocarbyl, for example, methoxy, ethoxy, propoxy, butoxy, pentoxy, methoxymethyl, ethoxymethyl, propoxymethyl, methoxyethyl, ethoxyethyl, propoxyethyl, methoxypropyl, ethoxypropyl, propoxypropyl, *N*-methylaminomethyl, *N*-methylaminoethyl, *N*-ethylaminoethyl, *N*,*N*-dimethylaminomethyl, *N*,*N*-dimethylaminoethyl, and *N*,*N*-diethylaminoethyl; the "aliphatic hydrocarbyl" moieties contained in the other groups are defined as above.

The term "C₃₋₁₂ cycloalkyl" refers to a saturated or unsaturated monovalent monocyclic or bicyclic hydrocarbon ring having 3-12 carbon atoms, and is preferably a "C₃₋₁₀ cycloalkyl". The term "C₃₋₁₀ cycloalkyl" refers to a saturated monovalent monocyclic or bicyclic hydrocarbon ring having 3, 4, 5, 6, 7, 8, 9 or 10 carbon atoms. The C₃₋₁₀ cycloalkyl may be a monocyclic hydrocarboyl such as cyclopropyl, cyclobutyl, cyclopentyl, cyclohexyl, cycloheptyl, cyclooctyl, cyclononyl and cyclodecyl, or may be a bicyclic hydrocarbyl such as a decahydronaphthalene ring.

The term "3-12 membered heterocyclyl" means a saturated or unsaturated monovalent monocyclic or bicyclic ring comprising 1-5 heteroatoms independently selected from N, O and S. The groups comprising heteroatoms are not aromatic, and the 3-12 membered heterocyclyl is preferably a "3-10 membered heterocyclyl". The term "3-10 membered heterocyclyl" means a saturated monovalent monocyclic or bicyclic ring comprising 1-5, preferably 1-3 heteroatoms selected from N, O and S. The heterocyclyl may be connected to the rest of the molecule through any of the carbon atoms or the nitrogen atom (if present). In particular, the heterocyclyl may include, but is not limited to: 4 membered rings such as azetidinyl and oxetanyl; 5 membered rings such as tetrahydrofuranyl, tetrahydrothienyl, dioxolyl, pyrrolidinyl, imidazolidinyl, pyrazolidinyl and pyrrolinyl; 6 membered rings such as tetrahydropyranyl, piperidinyl, morpholinyl, dithianyl, thiomorpholinyl, piperazinyl or trithianyl; or 7 membered rings such as diazepanyl. Optionally, the heterocyclyl may be benzo-fused. The heterocyclyl may be bicyclic, such as but not limited to a 5,5 membered ring such as a hexahydrocyclopenta[*c*]pyrrol-2(1*H*)-yl ring, or a 5,6 membered bicyclic ring such as a hexahydropyrrolo[1,2-*a*]pyrazin-2(1*H*)-yl ring. The ring comprising nitrogen atoms may be partially unsaturated, i.e., it may comprise one or more double bonds, such as but not limited to 2,5-dihydro-1*H*-pyrrolyl, 4*H*-[1,3,4]thiadiazinyl, 4,5-dihydrooxazolyl or 4*H*-[1,4]thiazinyl, or it may be benzo-fused, such as but not limited to dihydroisoquinolinyl. According to the present invention, the 3-12 membered heterocyclyl may be further selected from the following groups:

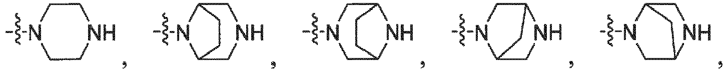

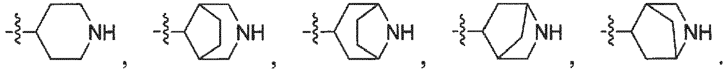

The term "C₆₋₂₀ aryl" refers to an aromatic or partially aromatic monovalent monocyclic, bicyclic or tricyclic hydrocarbon ring having 6-20 carbon atoms, and is preferably "C₆₋₁₄ aryl". The term "C₆₋₁₄ aryl" refers to preferably an aromatic or partially aromatic monovalent monocyclic, bicyclic or tricyclic hydrocarbon ring having 6, 7, 8, 9, 10, 11, 12, 13 or 14 carbon atoms ("C₆₋₁₄ aryl"), in particular a ring having 6 carbon atoms ("C₆ aryl"), such as phenyl; or a biphenyl, a ring having 9 carbon atoms ("C₉ aryl") such as indanyl or indenyl, a ring having 10 carbon atoms ("C₁₀ aryl") such as tetrahydronaphthyl, dihydronaphthyl or naphthyl, a ring having 13 carbon atoms ("C₁₃ aryl") such as fluorenyl, or a ring having 14 carbon atoms ("C₁₄ aryl") such as anthracenyl.

The term "5-14 membered heteroaryl" refers to an aromatic monovalent monocyclic, bicyclic or tricyclic ring, which has 5, 6, 7, 8, 9, 10, 11, 12, 13 or 14 ring atoms, in particular 5, 6, 9 or 10 carbon atoms, comprises 1-5, preferably 1-3 heteroatoms independently selected from N, O and S, and may be benzo-fused in each case. In particular, the heteroaryl is selected from thienyl, furyl, pyrrolyl, oxazolyl, thiazolyl, imidazolyl, pyrazolyl, isoxazolyl, isothiazolyl, oxadiazolyl, triazolyl, thiadiazolyl, thia-4*H*-pyrazolyl and the like and benzo derivatives thereof, such as benzofuranyl, benzothienyl, benzoxazolyl, benzoisoxazolyl, benzimidazolyl, benzotriazolyl, indazolyl, indolyl, and isoindolyl; or pyridinyl, pyridazinyl, pyrimidinyl, pyrazinyl, triazinyl and the like and benzo derivatives thereof, such as quinolyl, quinazolinyl, and isoquinolyl; or azocinyl, indolizinyl, purinyl and the like and benzo derivatives thereof; or cinnolinyl, phthalazinyl, quinazolinyl, quinoxalinyl, naphthyridinyl, pteridinyl, carbazolyl, acridinyl, phenazinyl, phenothiazinyl, phenoxazinyl, and the like.

Unless otherwise stated, the heterocyclyl or heteroaryl includes all possible isomeric forms thereof, e.g. positional isomers thereof. Accordingly, for some illustrative non-limiting examples, pyridinyl or pyridinylene includes pyridin-2-yl, pyridinylene-2-yl, pyridin-3-yl, pyridinylene-3-yl, pyridin-4-yl, and pyridinylene-4-yl; thienyl or thienylene includes thien-2-yl, thien-2-ylene, thien-3-yl, and thien-3-ylene.

Unless otherwise stated, the "C₃₋₁₂ cycloalkyl", "3-12 heterocyclyl", "C₆₋₂₀ aryl", and "5-14 membered heteroaryl" contained in other groups (e.g., *N*-containing 3-12 membered heterocycloalkyl or *N*-containing 5-14 membered heteroaryl) of the present invention are defined as above.

According to the structure, the compounds disclosed herein may be chiral and may therefore exist in various enantiomeric forms. These compounds may therefore exist in racemic or optically active form. The compounds disclosed herein or intermediates thereof may be separated into enantiomers by chemical or physical methods well known to those skilled in the art, or used in this form for synthesis. In the case of racemic amines, diastereoisomers are prepared from mixtures by reaction with optically active resolving agents. Examples of suitable resolving agents are optically active acids such as *R*- or *S*-tartaric acid, diacetyltartaric acid, dibenzoyltartaric acid, mandelic acid, malic acid, lactic acid, suitable *N*-protected amino acids (e.g., *N*-benzoylproline or *N*-benzenesulfonylproline) or various optically active camphorsulfonic acids. Enantiomeric resolution by chromatography can be advantageously performed with the aid of optically active resolving agents, such as dinitrobenzoylphenylglycine, cellulose triacetate or other carbohydrate derivatives or chirally derivatized methacrylate polymers immobilized on silica gel. Suitable eluents for this purpose are mixtures of solvent containing water or alcohol, for example, hexane/isopropanol/acetonitrile.

A pharmaceutically acceptable salt may be, for example, acid addition salts of the compounds disclosed herein having a nitrogen atom in the chain or ring with sufficient basicity, for example, acid addition salts formed with the following inorganic acids: hydrochloric acid, hydrofluoric acid, hydrobromic acid, hydroiodic acid, sulfuric acid, pyrosulfuric acid, phosphoric acid or nitric acid; hydrosulfates; or acid addition salts with the following organic acids: formic acid, acetic acid, acetoacetic acid, pyruvic acid, trifluoroacetic acid, propionic acid, butyric acid, hexanoic acid, heptanoic acid, undecanoic acid, lauric acid, benzoic acid, salicylic acid, 2-(4-hydroxybenzoyl)benzoic acid, camphoric acid, cinnamic acid, cyclopentanepropionic acid, digluconic acid, 3-hydroxy-2-naphthoic acid, nicotinic acid, pamoic acid, pectinic acid, peroxosulfuric acid, 3-phenylpropionic acid, picric acid, pivalic acid, 2-hydroxyethanesulfonic acid, itaconic acid, sulfamic acid, trifluoromethanesulfonic acid, dodecylsulfuric acid, ethanesulfonic acid, benzenesulfonic acid, *p*-toluenesulfonic acid, methanesulfonic acid, 2-naphthalenesulfonic acid, naphthalenedisulfonic acid, camphorsulfonic acid, citric acid, tartaric acid, stearic acid, lactic acid, oxalic acid, malonic acid, glycolic acid, valproic acid, succinic acid, malic acid, adipic acid, alginic acid, maleic acid, fumaric acid, D-gluconic acid, mandelic acid, ascorbic acid, glucoheptoic acid, glycerophosphoric acid, aspartic acid, sulfosalicylic acid, hemisulfuric acid, or thiocyanic acid.

In addition, another suitable pharmaceutically acceptable salt of the compound disclosed herein having sufficient acidity is an alkali metal salt (e.g., sodium salt or potassium salt), an alkaline earth metal salt (e.g., calcium salt or magnesium salt), an ammonium salt, or a salt formed with an organic base which provides a physiologically acceptable cation, for example a salt formed with: a sodium ion, a potassium ion, *N*-methylglucamine, dimethylglucamine, ethylglucamine, lysine, dicyclohexylamine, 1,6-hexanediamine, ethanolamine, glucosamine, meglumine, sarcosine, serinol, trihydroxymethylaminomethane, aminopropanediol, or 1-amino-2,3,4-butanetriol. As an example, the pharmaceutically acceptable salts include salts formed by the group -COOH with: a sodium ion, a potassium ion, a calcium ion, a magnesium ion, *N*-methylglucamine, dimethylglucamine, ethylglucamine, lysine, dicyclohexylamine, 1,6-hexanediamine, ethanolamine, glucosamine, meglumine, sarcosine, serinol, trishydroxymethylaminomethane, aminopropanediol, or 1-amino-2,3,4-butanetriol. In addition, the basic nitrogen-containing groups may be quaternized with the following agents: lower alkyl halides such as methyl, ethyl, propyl and butyl chlorides, bromides and iodides; dialkyl sulfates such as dimethyl sulfate, diethyl sulfate, dibutyl sulfate, and dipentyl sulfate; long chain halides such as decyl, lauryl, myristyl and stearyl chlorides, bromides and iodides; and aralkyl halides such as benzyl and phenethyl bromides. As an example, pharmaceutically acceptable salts include hydrochloride, sulfate, nitrate, bisulfate, hydrobromide, acetate, oxalate, citrate, mesylate, formate, meglumine, and the like.

Since the compound disclosed herein may have a plurality of salt-forming sites, the "pharmaceutically acceptable salt" includes not only a salt formed at 1 salt-forming site of the compound disclosed herein but also salts formed at 2, 3 or all of the salt-forming sites thereof. For this purpose, the molar ratio of the compound of formula (I) to a radical ion (anion) of an acid or a cation of a base required for salt formation may vary within a wide range, and may be, for example, 4:1 to 1:4, such as 3:1, 2:1, 1:1, 1:2, and 1:3.

According to the present invention, the pharmaceutically acceptable anions include anions selected from those generated by the ionization of inorganic or organic acids. According to the position and nature of the various substituents, the compound disclosed herein may also comprise one or more asymmetric centers. Asymmetric carbon atoms may exist in either the (R) or (*S*) configuration. When there is only one asymmetric center, a racemic mixture is generated, and when there are multiple asymmetric centers, a diastereoisomeric mixture is generated. In some cases, asymmetry may also exist due to hindered rotation about a particular bond, for example, the two substituted aromatic rings of a particular compound connected by the central bond may be asymmetric. Furthermore, the substituents may exist in cis- or trans-isomeric forms.

The compound disclosed herein also include all possible stereoisomers thereof, either in the form of a single stereoisomer or in the form of any mixture of the stereoisomers (e.g., R- or S-isomers, or E- or Z-isomers) in any proportion. Single stereoisomers (e.g., single enantiomers or single diastereoisomers) of the compound disclosed herein may be separated by any suitable method in the prior art (e.g., chromatography, particularly, e.g., chiral chromatography).

The term "tautomer" refers to functional isomers resulting from the rapid movement of an atom in a molecule between two positions. The compound disclosed herein may exhibit the tautomerism. Tautomeric compounds may exist in two or more interconvertible forms. Prototropic tautomers result from the migration of a covalently bonded hydrogen atom between two atoms. Tautomers generally exist in an equilibrium form. Trying to separate a single tautomer usually lead to a mixture, the physicochemical properties of which are consistent with the mixture of the compound. The position of the equilibrium depends on the chemical properties of the molecule. For example, in many aliphatic aldehydes and ketones such as acetaldehyde, the keto form predominates; whereas in phenol, the enol form predominates. The present invention comprises all tautomeric forms of the compound.

In the present invention, the compounds involved also include isotopically labeled compounds, which are identical to the compound of formula I, but have one or more atoms substituted by atoms with the atomic mass or mass number different from the atomic mass or mass number of those usually found in nature. Examples of isotopes that can be incorporated into the compound disclosed herein include isotopes of H, C, N, O, S, F and Cl, such as ²H, ³H, ¹³C, ¹¹C, ¹⁴C, ¹⁵N, ¹⁸O, ¹⁷O, ³²P, ³⁵S, ¹⁸F, and ³⁶Cl. The compound, the prodrug thereof, or the pharmaceutically acceptable salts thereof comprising the above isotopes and/or other isotopes of other atoms are within the scope of the present invention. Certain isotopically labeled compounds disclosed herein, e.g., those into which radioactive isotopes such as ³H and ¹⁴C are incorporated, are useful in drug and/or substrate tissue distribution assays. Tritium (i.e., ³H) and carbon 14 (i.e., ¹⁴C) isotopes are particularly preferred for their ease of preparation and detectability. Furthermore, substitution with heavier isotopes such as deuterium (i.e., ²H) may provide certain therapeutic advantages resulting from greater metabolic stability (e.g., increased *in vivo* half-life or reduced dosage requirement) and hence may be preferred in some cases. The compound disclosed herein as claimed may be particularly limited to substitution with deuterium or tritium. Furthermore, the lack of separate specification of a hydrogen in a substituent as the term deuterium or tritium does not mean that the deuterium or tritium is excluded, on the contrary, the deuterium or tritium can also be included. The term "effective amount" or "therapeutically effective amount" refers to an amount of the compound disclosed herein sufficient to effect the intended use, including but not limited to the treatment of diseases or conditions described herein. The therapeutically effective amount may vary depending on the following factors: the intended use (*in vitro* or *in vivo*), or the subject and diseases or conditions being treated, such as weight and age of the subject, severity of the diseases or conditions and mode of administration, etc., which can be readily determined by one of ordinary skill in the art. The specific dosage will vary depending on the following factors: the particular compound selected, the dosage regimen to be followed, whether to administer in combination with other compounds, the schedule of administration, the tissue to be administered and the physical delivery system carried. For diseases or conditions described herein (e.g., pain), an effective amount includes an amount sufficient to alleviate or relieve to some extent the associated symptoms.

The term "excipient" or carrier, refers to an additive in a pharmaceutical preparation, besides the main drug. Preservatives, antioxidants, flavoring agents, fragrances, cosolvents, emulsifiers, solubilizers, tonicity adjusting agents, colorants and the like in liquid preparations can be referred to as excipients. Examples of suitable pharmaceutically acceptable excipients are described in Excipients and their use in injectable products. PDA J Pharm Sci TechnoL, Jul-Aug 1997, 51:166-171; and Excipient Selection In Parenteral Formulation Development, Pharma Times, Mar 2013, 45(3):65-77, which are incorporated herein by reference in their entirety. Examples of typical pharmaceutically acceptable excipients include: saccharides such as lactose, sucrose, mannitol, and sorbitol; starches, such as corn starch, tapioca starch and potato starch; cellulose and its derivatives, such as sodium carboxymethylcellulose, ethyl cellulose and methyl cellulose; calcium phosphates such as dicalcium phosphate and tricalcium phosphate; sodium sulfate; calcium sulfate; polyvinylpyrrolidone; polyvinyl alcohol; stearic acid; alkaline earth metal stearate, such as magnesium stearate and calcium stearate; stearic acid; vegetable oils, such as peanut oil, cottonseed oil, sesame oil, olive oil and corn oil; nonionic, cationic and anionic surfactants; glycol polymer; fatty alcohols, and the like.

The term "solvate" refers to forms of the compound disclosed herein that form complexes in the solid or liquid state by coordination with solvent molecules. Hydrate is a particular form of the solvate in which the coordination is performed with water. In the present invention, the preferred solvate is a hydrate. Further, pharmaceutically acceptable solvates (hydrates) of the compound of formula I disclosed herein refer to co-crystals and clathrates of the compound of formula I with one or more water molecules or other solvents in stoichiometric amounts. Solvents useful for solvates include, but are not limited to: water, methanol, ethanol, ethylene glycol, propylene glycol, ethanolamine, and acetic acid.

The term "bioavailability" refers to the relative amount of a drug circulated in the systemic blood following extravascular administration. In pharmacology, it means that the dose portion of the drug administered can achieve the systemic circulation and is a pharmacokinetic property of the drug.

### Beneficial Effects

1) The compound disclosed herein is relatively stable *in vitro,* and can be quickly converted into *trans*-8-methyl-*N*-vanillyl-6-nonenamide *in vivo* and exerts the corresponding TPV1 agonist activity. Therefore, the compound disclosed herein can be used as a prodrug of *trans*-8-methyl-*N*-vanillyl-6-nonenamide, and has better druggability and higher application value than *trans*-8-methyl-*N*-vanillyl-6-nonenamide.
2) Compared with *trans*-8-methyl-*N*-vanillyl-6-nonenamide, which has extremely poor water solubility (solubility: 0.00841 mg/mL), the compound disclosed herein has remarkably excellent water solubility.
3) The compound disclosed herein is a novel amino acid derivative of *trans*-8-methyl-*N*-vanillyl-6-nonenamide, has remarkably smaller irritation, better preparation safety and stability and the like than *trans*-8-methyl-*N*-vanillyl-6-nonenamide.

### BRIEF DESCRIPTION OF THE DRAWINGS

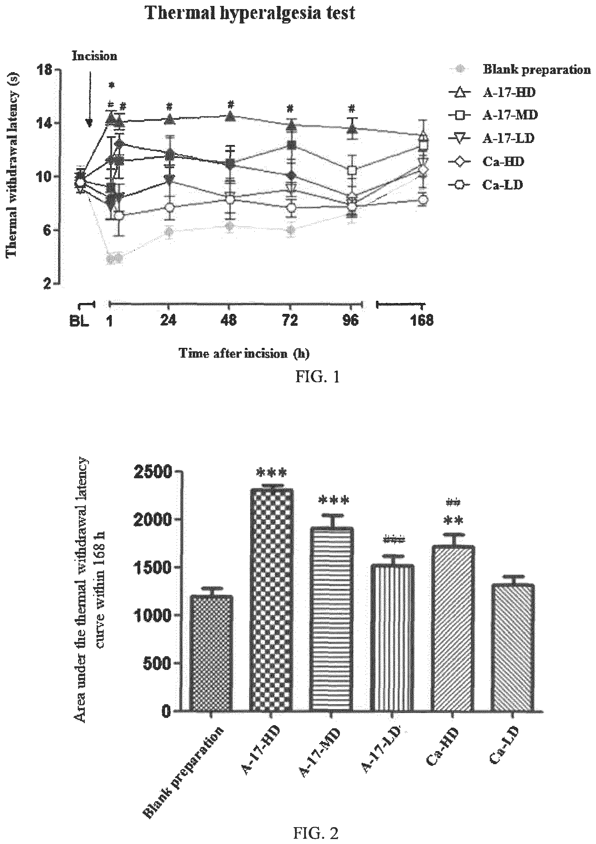

FIG. 1. Effect of compound A-17 administrated by subcutaneous injection at incision site on thermal hyperalgesia in rats as models of pain after incision. BL indicates the thermal withdrawal latency (TWL) before incision, and the black filled dot indicates a statistical difference (P < 0.05) in corresponding time points between each treatment group and the blank preparation group. ^{∗} indicates a statistical difference (P < 0.05) between the A-17-HD group and the A-17-MD group; # indicates a statistical difference (P < 0.05) between the A-17-HD group and the A-17-LD group. Ca indicates *trans*-8-methyl-*N*-vanillyl-6-nonenamide.
FIG. 2. Effect of compound A-17 administrated by subcutaneous injection at incision site on overall thermal hyperalgesia (AUC) over 7 days in rats as models of pain after incision. A statistical difference compared with the blank preparation group, ^{∗∗}P < 0.01, ^{∗∗∗}P < 0.001; a statistical difference compared with A-17-HD, ##P < 0.01, ###P < 0.001. Ca indicates *trans-*8-methyl-*N-*vanillyl-6-nonenamide.

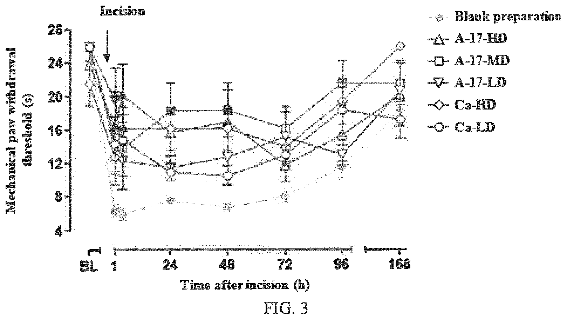

FIG. 3. Effect of compound A-17 administrated by subcutaneous injection at incision site on mechanical allodynia in rats as models of pain after incision. BL indicates the paw withdrawal threshold (PWT) before incision, and the black filled dot indicates a statistical difference (P < 0.05) in corresponding time points between each treatment group and the blank preparation group. Ca indicates *trans*-8-methyl-*N*-vanillyl-6-nonenamide.

### DETAILED DESCRIPTION

The technical solution of the present invention will be further illustrated in detail with reference to the following specific examples. It should be understood that the following examples are merely exemplary illustration and explanation of the present invention, and should not be construed as limiting the scope of protection of the present invention. All techniques implemented based on the aforementioned contents of the present invention are encompassed within the scope of protection of the present invention.

Unless otherwise stated, the starting materials and reagents used in the following examples are all commercially available or can be prepared by known methods.

Unless otherwise stated, it should be understood by those skilled in the art that pharmaceutically acceptable salts can be prepared from the compound using conventional acid or base addition procedures, and the salts of the compound can be converted into free products under acid or base addition conditions.

### Example 1. Synthesis of (E)-2-methoxy-4-((8-methylnon-6-enamido)methyl)phenyldiethylglycine hydrochloride (compound A-1)

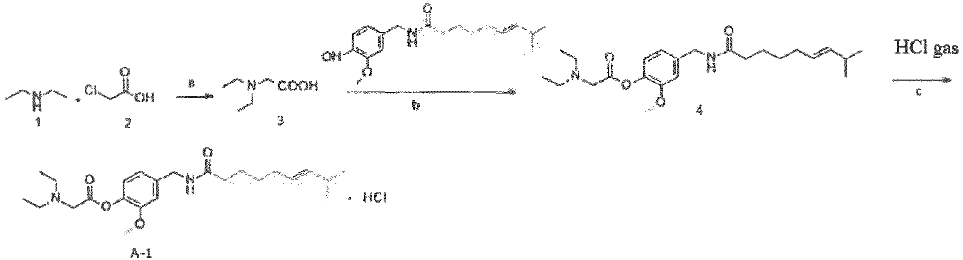

Reaction reagents and conditions: (a) ethylenediamine, chloroacetic acid, rt, 6 h, 89.3%; (b) *trans*-8-methyl-*N*-vanillyl-6-nonenamide, EDCI, DMAP, DCM, rt, 3 h, 43.6%; (c) sodium chloride, concentrated sulfuric acid, rt, 1 h, 95.3%.

### Synthesis of ethylglycine (compound 3)

Compound 1 (10 mL, 7 g, 95.7 mmol) was added to a reaction flask and cooled to 5 °C in an ice bath. The reaction system was slowly added with compound 2 (1.29 g, 13.6 mmol) in portions, warmed to 30 °C, and stirred for 6 h until the reaction system turned yellow and finally reddish brown. The reaction solution was filtered in vacuum, and the filter cake was washed with ethyl acetate. The filtrate was concentrated by rotary evaporation to give a reddish-brown oil (1.6 g, 89.3% yield).

Synthesis of (*E*)-2-methoxy-4-((8-methylnon-6-enamido) methyl) phenyldiethylglycine (compound 4) To a solution of compound 3 (1.28 g, 9.82 mmol) in dichloromethane were added 1-(3-dimethylaminopropyl)-3-ethylcarbodiimide hydrochloride (1.03 g, 5.401 mmol) and 4-dimethylaminopyridine (0.059 g, 0.491 mmol). The reaction system was stirred for 30 min, added with *trans*-8-methyl-*N*-vanillyl-6-nonenamide (1.5 g, 4.91 mmol), and stirred at room temperature for 3 h. Then the reaction system was washed with hydrochloric acid (2 N) three times, washed with water three times, dried over anhydrous sodium sulfate, and filtered. The filtrate was concentrated under reduced pressure, and the crude product was purified by column chromatography to give an oil (about 0.89 g, 43.6% yield).

### Synthesis of (E)-2-methoxy-4-((8-methylnon-6-enamido) methyl) phenyldiethylglycine hydrochloride (compound A-1)

Compound 4 (0.89 g, 2.127 mmol) was dissolved in a certain amount of ethyl acetate. The reaction system was purged with dry HCl gas generated by the reaction of sodium chloride with concentrated sulfuric acid, with the pH maintained at 3-4, reacted at room temperature for 1 h, and filtered in vacuum to give a white solid (0.92 g, 95.3% yield). ¹H NMR (300 MHz, DMSO-d₆): δ 10.62 (s, 1H), 8.47 (t, J = 5.9 Hz, 1H), 7.10 (dd, J = 16.3, 4.9 Hz, 2H), 6.86 (dd, J = 8.2, 1.7 Hz, 1H), 5.42 - 5.25 (m, 2H), 4.52 (d, J = 3.9 Hz, 2H), 4.26 (d, J = 5.9 Hz, 2H), 3.76 (s, 3H), 3.27 (dd, J = 7.0, 3.9 Hz, 4H), 2.24 - 2.10 (m, 3H), 1.94 (dd, J = 12.9, 6.9 Hz, 2H), 1.57 - 1.45 (m, 2H), 1.27 (t, J = 7.2 Hz, 8H), 0.92 (d, J = 6.7 Hz, 6H). HRMS (ESI): m/z, calcd for C₂₄H₃₉N₂O₄ [M + H]⁺, 419.2904; found: 419.2886.

### Example 2. Synthesis of (E)-2-methoxy-4-((8-methylnon-6-enamidoyl)methyl)phenyl N-ethyl-N-isopropylglycine hydrochloride (compound A-2)

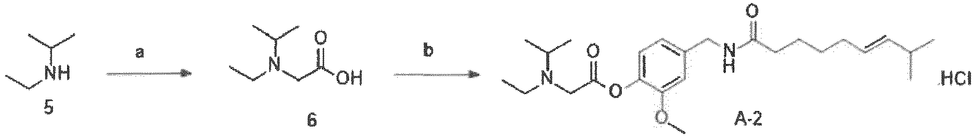

Reaction reagents and conditions: (a) chloroacetic acid, DCM, rt, 3 h, 78.3%; (b) *trans*-8-methyl-*N*-vanillyl-6-nonenamide, EDCI, DMAP, sodium chloride, concentrated sulfuric acid, DCM, EA, rt, 3 h, 59.3%.

### Synthesis of N-ethyl-N-isopropylglycine (compound 6)

Compound 5 (2.3 g, 26.47 mmol) was dissolved in a certain amount of dichloromethane. The reaction system was cooled to 5 °C in an ice bath, slowly added with chloroacetic acid (0.5 g, 5.29 mmol), and reacted at room temperature for 3 h. Then the reaction system was concentrated under reduced pressure to remove dichloromethane, and the residue was added with a certain amount of ethyl acetate, and filtered in vacuum to remove the precipitated salts. The filtrate was concentrated by rotary evaporation to give an oil (compound 6).

### Synthesis of (E)-2-methoxy-4-((8-methylnon-6-enamidoyl) methyl) phenyl N-ethyl-N-isopropylglycine hydrochloride (compound A-2)

Compound 6 (0.48 g, 3.274 mmol) was dissolved in a certain amount of dichloromethane. The reaction system was cooled to 5 °C in an ice bath, slowly added with *trans*-8-methyl-*N*-vanillyl-6-nonenamide (0.5 g, 1.64 mmol), 1-(3-dimethylaminopropyl)-3-ethylcarbodiimide hydrochloride (0.48 g, 2.45 mmol) and 4-dimethylaminopyridine (0.04 g, 0.75 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-2). HRMS (ESI): m/z, calcd for C₂₅H₄₁N₂O₄ [M + H]⁺, 433.3061; found: 433.3057.

### Example 3. Synthesis of (E)-2-methoxy-4-((8-methylnon-6-enamido) methyl) phenyl N-ethyl-N-propylglycine hydrochloride (compound A-3)

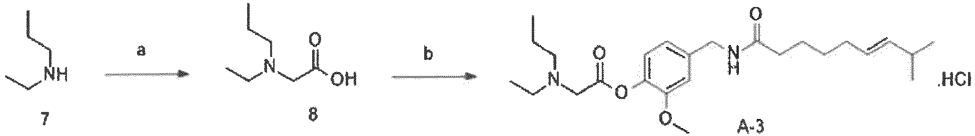

Reaction reagents and conditions: (a) chloroacetic acid, DCM, rt, 3 h, 85.3%; (b) *trans*-8-methyl-*N*-vanillyl-6-nonenamide, EDCI, DMAP, sodium chloride, concentrated sulfuric acid, DCM, EA, rt, 3 h, 48.6%.

### Synthesis of N-ethyl-N-propylglycine (compound 8)

Compound 7 (3.9 g, 26.9 mmol) was dissolved in a certain amount of dichloromethane. The reaction system was cooled to 5 °C in an ice bath, slowly added with chloroacetic acid (0.5 g, 5.29 mmol), and reacted at room temperature for 3 h. Then the reaction system was concentrated under reduced pressure to remove dichloromethane, and the residue was added with a certain amount of ethyl acetate, and filtered in vacuum to remove the precipitated salts. The filtrate was concentrated by rotary evaporation to give an oil (compound 8).

### Synthesis of (E)-2-methoxy-4-((8-methylnon-6-enamido) methyl) phenyl N-ethyl-N-propylglycinate hydrochloride (compound A-3)

Compound 8 (0.11 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-3). HRMS (ESI): m/z, calcd for C₂₅H₄₁N₂O₄ [M + H]⁺, 433.3061; found: 433.3050.

### Example 4. Synthesis of (E)-2-methoxy-4-((8-methylnon-6-enamido) methyl) phenyl N-butyl-N-ethylglycine hydrochloride (compound A-4)

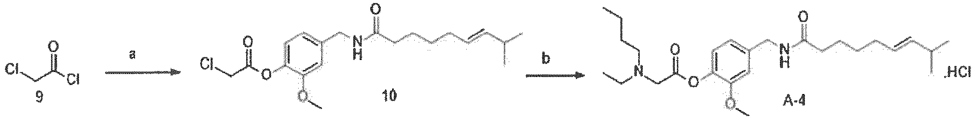

Reaction reagents and conditions: (a) *trans*-8-methyl-*N*-vanillyl-6-nonenamide, triethylamine, DCM, rt, 1 h, 90.3%; (b) *N*-ethyl butylamine, sodium chloride, concentrated sulfuric acid, acetonitrile, EA, rt, 4 h, 78.2%.

### Synthesis of (E)-2-methoxy-4-((8-methylnon-6-enamido) methyl) phenyl 2-chloroacetate (compound 10)

*Trans*-8-methyl-*N*-vanillyl-6-nonenamide (1 g, 3.274 mmol) was dissolved in a certain amount of dichloromethane. The reaction system was cooled to 0 °C in an ice bath, added with triethylamine (0.66 g, 6.548 mmol), slowly added dropwise with chloroacetyl chloride (0.55 g, 4.911 mmol), and reacted at room temperature for 1 h. Then the reaction system was concentrated under reduced pressure to remove excess dichloromethane to give an oil (compound 10).

### Synthesis of (E)-2-methoxy-4-((8-methylnon-6-enamido) methyl) phenyl N-butyl-N-ethylglycinate hydrochloride (compound A-4)

Compound 10 (0.3 g, 0.79 mmol) was dissolved in a certain amount of acetonitrile. The reaction system was cooled to 5 °C in an ice bath, slowly added with *N*-ethyl butylamine (0.24 g, 2.36 mmol), and stirred at room temperature for 3 h. Then the reaction system was concentrated under reduced pressure to removed acetonitrile to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-4). HRMS (ESI): m/z, calcd for C₂₆H₄₃N₂O₄ [M + H]⁺, 447.3217; found: 433.3222.

### Example 5. Synthesis of (E)-2-methoxy-4-((8-methylnon-6-enamido)methyl)phenyldimethylglycine hydrochloride (compound A-5)

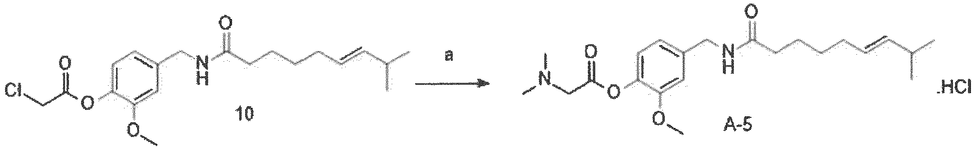

Reaction reagents and conditions: (a) dimethylamine, sodium chloride, concentrated sulfuric acid, acetonitrile, EA, rt, 4 h, 80.3%.

### Synthesis of (E)-2-methoxy-4-((8-methylnon-6-enamido)methyl)phenyldimethylglycine hydrochloride (compound A-5)

Compound 10 (0.3 g, 0.79 mmol) was dissolved in a certain amount of acetonitrile. The reaction system was cooled to 5 °C in an ice bath, slowly added with dimethylamine (0.11 g, 2.36 mmol), and stirred at room temperature for 3 h. Then the reaction system was concentrated under reduced pressure to removed acetonitrile to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-5). HRMS (ESI): m/z, calcd for C₂₂H₃₅N₂O₄ [M + H]⁺, 391.2591; found: 351.2592.

### Example 6. Synthesis of (E)-2-methoxy-4-((8-methylnon-6-enamido)methyl)phenyl 2-(pyrrolidin-1-yl)acetic acid hydrochloride (compound A-6)

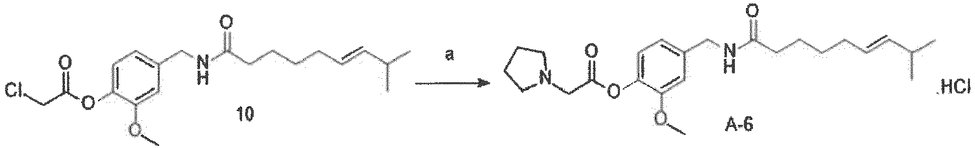

Reaction reagents and conditions: (a) pyrrolidine, sodium chloride, concentrated sulfuric acid, acetonitrile, EA, rt, 4 h, 75.4%.

### Synthesis of (E)-2-methoxy-4-((8-methylnon-6-enamido)methyl)phenyl 2-(pyrrolidin-1-yl)acetic acid hydrochloride (compound A-6)

Compound 10 (0.3 g, 0.79 mmol) was dissolved in a certain amount of acetonitrile. The reaction system was cooled to 5 °C in an ice bath, slowly added with pyrrolidine (0.17 g, 2.36 mmol), and stirred at room temperature for 3 h. Then the reaction system was concentrated under reduced pressure to removed acetonitrile to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-6). HRMS (ESI): m/z, calcd for C₂₄H₃₇N₂O₄ [M + H]⁺, 417.2748; found: 417.2740.

### Example 7. Synthesis of (E)-2-methoxy-4-((8-methylnon-6-enamido)methyl)phenyl 2-(methyl-1-azanaphthyl)propionic acid hydrochloride (compound A-7)

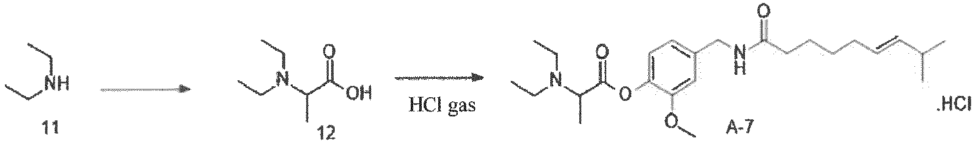

Reaction reagents and conditions: (a) chloropropionic acid, DCM, rt, 3 h, 81.2%; (b) *trans*-8-methyl-*N*-vanillyl-6-nonenamide, EDCI, DMAP, sodium chloride, concentrated sulfuric acid, DCM, EA, rt, 3 h, 49.6%.

### Synthesis of N,N-diethylalanine (compound 12)

Compound 11 (1.97 g, 26.9 mmol) was dissolved in a certain amount of dichloromethane. The reaction system was cooled to 5 °C in an ice bath, slowly added with chloroacetic acid (0.5 g, 5.29 mmol), and reacted at room temperature for 3 h. Then the reaction system was concentrated under reduced pressure to remove dichloromethane, and the residue was added with a certain amount of ethyl acetate, and filtered in vacuum to remove the precipitated salts. The filtrate was concentrated by rotary evaporation to give an oil (compound 12).

### Synthesis of (E)-2-methoxy-4-((8-methylnon-6-enamido)methyl)phenyldiethylalanine hydrochloride (compound A-7)

Compound 12 (0.11 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-7). HRMS (ESI): m/z, calcd for C₂₅H₄₁N₂O₄ [M + H]⁺, 433.3061; found: 433.3057.

### Example 8. Synthesis of (E)-2-methoxy-4-((8-methylnon-6-enamido)methyl)phenyldiethyl-L-valine hydrochloride (compound A-8)

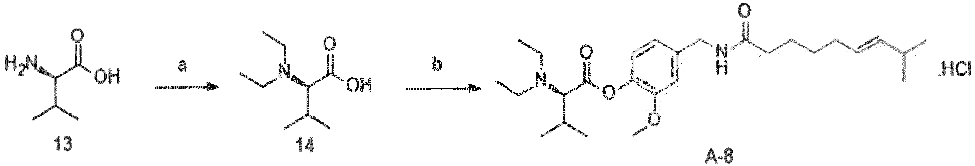

Reaction reagents and conditions: (a) NaBH₃CN, CH₃CHO, H₂O, MeOH, rt, 5 h, 76.2%; (b) *trans*-8-methyl-*N*-vanillyl-6-nonenamide, EDCI, DMAP, sodium chloride, concentrated sulfuric acid, DCM, EA, rt, 3 h, 53.4%.

### Synthesis of N,N-diethyl-L-valine (compound 14)

L-valine (0.35 g, 3 mmol) was dissolved in a certain amount of mixed solution of water and methanol. The reaction system was cooled to 0 °C in an ice bath, slowly added with sodium cyanoborohydride (0.48 g, 7.3 mmol), slowly added dropwise with an aqueous acetaldehyde solution (1.44 mL, 14.26 mmol) after the solution was clear, and stirred at room temperature for 5 h. Then the reaction system was added with a certain amount of concentrated hydrochloric acid to adjust the pH to about 1.5, and concentrated under reduced pressure to remove the solvent to give an oil (compound 14), which was directly used in the next step.

### Synthesis of (E)-2-methoxy-4-((8-methylnon-6-enamido)methyl)phenyldiethyl-L-valine hydrochloride (compound A-8)

Compound 14 (0.14 g, 0.79 mmol) was dissolved in a certain amount of dichloromethane. The reaction system was cooled to 5 °C in an ice bath, slowly added with *trans-*8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-8). HRMS (ESI): m/z, calcd for C₂₇H₄₅N₂O₄ [M + H]⁺, 461.3374; found: 461.3371.

### Example 9. Synthesis of (E)-2-methoxy-4-((8-methylnon-6-enamido)methyl)phenyldiethyl-L-leucine hydrochloride (compound A-9)

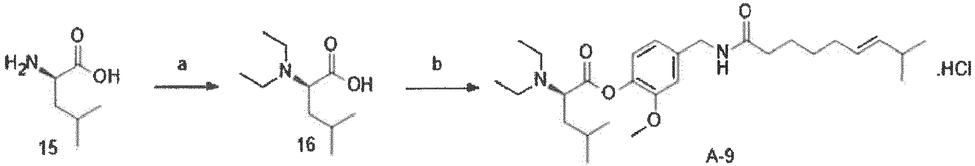

Reaction reagents and conditions: (a) NaBH₃CN, CH₃CHO, H₂O, MeOH, rt, 5 h, 81.2%; (b) *trans*-8-methyl-*N*-vanillyl-6-nonenamide, EDCI, DMAP, sodium chloride, concentrated sulfuric acid, DCM, EA, rt, 3 h, 53.8%.

### Synthesis of N,N-diethyl-L-leucine (compound 16)

L-leucine (0.39 g, 3 mmol) was dissolved in a certain amount of mixed solution of water and methanol. The reaction system was cooled to 0 °C in an ice bath, slowly added with sodium cyanoborohydride (0.48 g, 7.3 mmol), slowly added dropwise with an aqueous acetaldehyde solution (1.44 mL, 14.26 mmol) after the solution was clear, and stirred at room temperature for 5 h. Then the reaction system was added with a certain amount of concentrated hydrochloric acid to adjust the pH to about 1.5, and concentrated under reduced pressure to remove the solvent to give an oil (compound 16), which was directly used in the next step.

### Synthesis of (E)-2-methoxy-4-((8-methylnon-6-enamido)methyl)phenyldiethyl-L-leucine hydrochloride (compound A-9)

Compound 16 (0.15 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-9). HRMS (ESI): m/z, calcd for C₂₈H₄₇N₂O₄ [M + H]⁺, 475.3530; found: 475.3497.

### Example 10. Synthesis of 2-methoxy-4-((8-methylnon-6-enamido)methyl)phenyldiethyl-L-isoleucine hydrochloride (compound A-10)

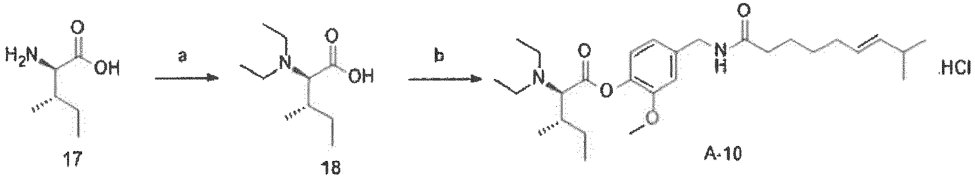

Reaction reagents and conditions: (a) NaBH₃CN, CH₃CHO, H₂O, MeOH, rt, 5 h, 83.2%; (b) *trans*-8-methyl-*N*-vanillyl-6-nonenamide, EDCI, DMAP, sodium chloride, concentrated sulfuric acid, DCM, EA, rt, 3 h, 60.5%.

### Synthesis of N,N-diethyl-L-isoleucine (compound 18)

L-leucine (0.39 g, 3 mmol) was dissolved in a certain amount of mixed solution of water and methanol. The reaction system was cooled to 0 °C in an ice bath, slowly added with sodium cyanoborohydride (0.48 g, 7.3 mmol), slowly added dropwise with an aqueous acetaldehyde solution (1.44 mL, 14.26 mmol) after the solution was clear, and stirred at room temperature for 5 h. Then the reaction system was added with a certain amount of concentrated hydrochloric acid to adjust the pH to about 1.5, and concentrated under reduced pressure to remove the solvent to give an oil (compound 18), which was directly used in the next step.

### Synthesis of (E)-2-methoxy-4-((8-methylnon-6-enamido)methyl)phenyldiethyl-L-leucine hydrochloride (compound A-10)

Compound 18 (0.15 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-10). HRMS (ESI): m/z, calcd for C₂₈H₄₇N₂O₄ [M + H]⁺, 475.3530; found: 475.3502.

### Example 11. Synthesis of (E)-2-methoxy-4-((8-methylnon-6-enamido)methyl)phenyl N-ethyl-N-methylalanine hydrochloride (compound A-11)

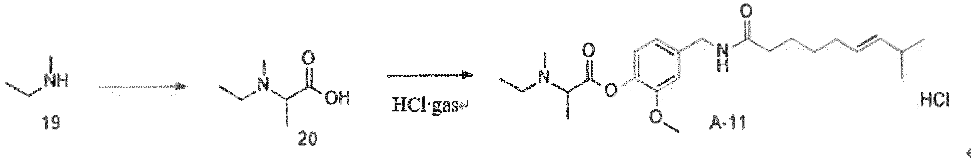

Reaction reagents and conditions: (a) chloropropionic acid, DCM, rt, 3 h, 75.3%; (b) *trans*-8-methyl-*N-*vanillyl-6-nonenamide*,* EDCI, DMAP, sodium chloride, concentrated sulfuric acid, DCM, EA, rt, 3 h, 70.2%.

### Synthesis of N-ethyl methyl alanine (compound 20)

Compound 19 (1.59 g, 26.9 mmol) was dissolved in a certain amount of dichloromethane. The reaction system was cooled to 5 °C in an ice bath, slowly added with chloroacetic acid (0.5 g, 5.29 mmol), and reacted at room temperature for 3 h. Then the reaction system was concentrated under reduced pressure to remove dichloromethane, and the residue was added with a certain amount of ethyl acetate, and filtered in vacuum to remove the precipitated salts. The filtrate was concentrated by rotary evaporation to give an oil (compound 20).

### Synthesis of (E)-2-methoxy-4-((8-methylnon-6-enamido)methyl)phenyl N-ethyl-N-methylalanine hydrochloride (compound A-11)

Compound 20 (0.10 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-11). HRMS (ESI): m/z, calcd for C₂₄H₃₉N₂O₄ [M + H]⁺, 419.2904; found: 419.2901.

### Example 12. Synthesis of (E)-2-methoxy-4-((8-methylnon-6-enamido)methyl)phenyl N-ethyl-N-methylglycine hydrochloride (compound A-12)

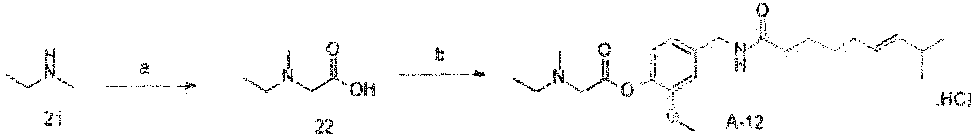

Reaction reagents and conditions: (a) chloroacetic acid, DCM, rt, 3 h, 80.2%; (b) *trans*-8-methyl-*N-*vanillyl-6-nonenamide*,* EDCI, DMAP, sodium chloride, concentrated sulfuric acid, DCM, EA, rt, 3 h, 60.1%.

### Synthesis of N-ethyl-N-methylglycine (compound 22)

Compound 21 (3.18 g, 53.80 mmol) was dissolved in a certain amount of dichloromethane. The reaction system was cooled to 5 °C in an ice bath, slowly added with chloroacetic acid (1 g, 10.58 mmol), and reacted at room temperature for 3 h. Then the reaction system was concentrated under reduced pressure to remove dichloromethane, and the residue was added with a certain amount of ethyl acetate, and filtered in vacuum to remove the precipitated salts. The filtrate was concentrated by rotary evaporation to give an oil (compound 22).

### Synthesis of (E)-2-methoxy-4-((8-methylnon-6-enamido)methyl)phenyl N-ethyl-N-methylglycinate hydrochloride (compound A-12)

Compound 22 (0.09 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-12). HRMS (ESI): m/z, calcd for C23H37N2O4 [M + H]⁺, 405.2748; found: 405.2742.

### Example 13. Synthesis of (E)-2-methoxy-4-(8-methylnonanamide)phenylglycine hydrochloride (compound A-13)

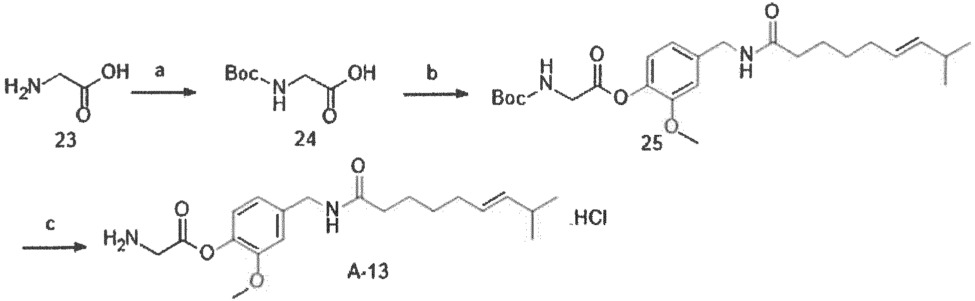

Reaction reagents and conditions: (a) (Boc)₂O, TEA, H₂O, 1 h, 80.3%; (b) *trans*-8-methyl-*N*-vanillyl-6-nonenamide, EDCI, DMAP, DCM, 2 h, 60.5%; (c) sodium chloride, concentrated sulfuric acid, DCM, rt, 0.5 h, 86.1%.

### Synthesis of (tert-butoxycarbonyl)glycine (compound 24)

Compound 23 (2 g, 26.64 mmol) was dissolved in a certain amount of water. The reaction system was slowly added with di-*tert*-butyl methyl dicarbonate (8.73 g, 39.96 mmol) and triethylamine (8.09 g, 79.92 mmol), and reacted at room temperature for 1 h. Then the reaction system was adjusted to pH 4-5 with hydrochloric acid (2 M), and extracted with ethyl acetate to give a pale yellow oil (compound 24, 3.74 g).

### Synthesis of (E)-2-methoxy-4-((8-methylnon-6-yl)methyl)pheny](tert-butoxycarbonyl)glycine (compound 25)

Compound 24 (0.31 g, 1.639mmol) was dissolved in a certain amount of dichloromethane. The reaction system was cooled to 5 °C in an ice bath, slowly added with *trans*-8-methyl-*N*-vanillyl-6-nonenamide (0.5 g, 1.637 mmol), 1-(3-dimethylaminopropyl)-3-ethylcarbodiimide hydrochloride (0.48 g, 2.45 mmol) and 4-dimethylaminopyridine (0.04 g, 0.325 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil (compound 25).

### Synthesis of (E)-2-methoxy-4-(8-methylnonanamide)phenylglycine hydrochloride (compound A-13)

The oil (compound 25) was dissolved in a certain amount of ethyl acetate. The reaction system was purged with HCl gas generated by the reaction of sodium chloride with concentrated sulfuric acid until the pH was adjusted to 3-4, stirred at room temperature for 0.5 h, and filtered in vacuum to give a white solid (compound A-13). 1H NMR (300 MHz, MeOD) δ 7.07 (d, J = 8.0 Hz, 2H), 6.90 (dd, J = 8.2, 1.6 Hz, 1H), 5.43-5.28 (m, 2H), 4.37 (s, 2H), 4.15 (s, 2H), 3.83 (d, J = 4.5 Hz, 3H), 2.23 (dt, J = 12.6, 6.3 Hz, 3H), 2.00 (dd, J = 12.5, 7.2 Hz, 2H), 1.70-1.58 (m, 2H), 1.43-1.31 (m, 2H), 0.96 (d, J = 6.7 Hz, 6H). HRMS (ESI): m/z, calcd for C₂₀H₃₀N₂O₄ [M + H]⁺, 363.2278; found: 363.2273.

### Example 14. Synthesis of (E)-2-methoxy-4-(8-methylnonanamide)phenyl 2-(piperidin-1-yl)acetic acid hydrochloride (compound A-14)

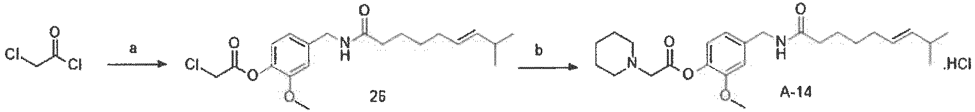

Reaction reagents and conditions: (a) *trans*-8-methyl-*N*-vanillyl-6-nonenamide, triethylamine, DCM, rt, 1 h, 90.3%; (b) piperidine, sodium chloride, concentrated sulfuric acid, DCM, EA, rt, 4 h, 75.4%.

### Synthesis of (E)-2-methoxy-4-((8-methylnon-6-enamido)methyl)phenyl 2-chloroacetate (compound 26)

*Trans*-8-methyl-*N*-vanillyl-6-nonenamide (1 g, 3.274 mmol) was dissolved in a certain amount of dichloromethane. The reaction system was cooled to 0 °C in an ice bath, added with triethylamine (0.66 g, 6.548 mmol), slowly added dropwise with chloroacetyl chloride (0.55 g, 4.911 mmol), and reacted at room temperature for 1 h. Then the reaction system was concentrated under reduced pressure to remove excess dichloromethane to give an oil (compound 26).

### Synthesis of (E)-2-methoxy-4-(8-methylnonanamide)phenyl 2-(piperidin-1-yl)acetic acid hydrochloride (compound A-14)

Compound 26 (0.3 g, 0.79 mmol) was dissolved in a certain amount of DCM. The reaction system was cooled to 5 °C in an ice bath, slowly added with piperidine (0.17 g, 2.36 mmol), and stirred at room temperature for 3 h. Then the reaction system was concentrated under reduced pressure to removed acetonitrile to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-14). 1H NMR (300 MHz, DMSO) δ 10.64 (s, 1H), 8.47 (t, J = 5.9 Hz, 1H), 7.18-7.02 (m, 2H), 6.86 (d, J = 8.4 Hz, 1H), 5.42-5.25 (m, 2H), 4.53 (s, 2H), 4.27 (d, J = 5.9 Hz, 2H), 3.77 (s, 3H), 3.55 (s, 2H), 3.08 (s, 2H), 2.19 (dt, J = 19.9, 7.1 Hz, 3H), 1.94 (dd, J = 12.9, 6.7 Hz, 2H), 1.88-1.66 (m, 5H), 1.57-1.46 (m, 2H), 1.30 (dt, J = 19.2, 9.8 Hz, 3H), 0.93 (d, J = 6.7 Hz, 6H). HRMS (ESI): m/z, calcd for C25H38N2O4 [M + H]⁺, 431.2904; found: 431.2464.

### Example 15. Synthesis of (E)-2-methoxy-4-(8-methylnonanamide)phenyl 2-(dimethylamino)-2-phenylacetic acid hydrochloride (compound A-15)

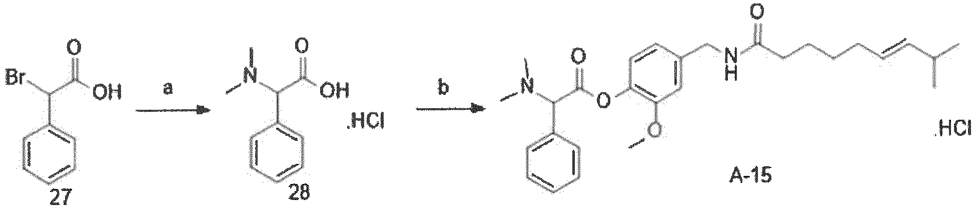

Reaction reagents and conditions: (a) dimethylamine, hydrochloric acid, acetone, rt, 1 h, 78.3%; (b) *trans*-8-methyl-*N*-vanillyl-6-nonenamide, EDCI, DMAP, sodium chloride, concentrated sulfuric acid, DCM, EA, rt, 3 h, 68.2%.

### Synthesis of 2-(dimethylamino)-2-phenylacetic acid hydrochloride (compound 28)

Compound 27 (2 g, 9.3 mmol) was dissolved in a certain amount of acetone solution. The reaction system was cooled to 5 °C in an ice bath, slowly added with dimethylamine (0.84 g, 18.6 mmol) and stirred at room temperature for 1 h. Then the reaction system was added with hydrochloric acid to adjust the pH to 3-4, and concentrated by rotary evaporation to remove the solvent. The residue was re-crystallized from ethanol to give a white solid (compound 28).

### Synthesis of (E)-2-methoxy-4-(8-methylnonanamide)phenyl 2-(dimethylamino)-2-phenylacetic acid hydrochloride (compound A-15)

Compound 28 (0.17 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-15). 1H NMR (300 MHz, DMSO) δ 8.43 (t, J = 5.9 Hz, 1H), 7.69 (s, 2H), 7.58 (d, J = 2.9 Hz, 2H), 7.52 (d, J = 3.9 Hz, 1H), 7.00 (d, J = 8.2 Hz, 2H), 6.82 (d, J = 7.8 Hz, 1H), 5.85 (s, 1H), 5.41-5.24 (m, 2H), 4.24 (d, J = 5.8 Hz, 2H), 3.64 (s, 3H), 3.00 (s, 2H), 2.89 (d, J = 6.5 Hz, 2H), 2.59 (s, 2H), 2.16 (dt, J = 14.6, 7.0 Hz, 3H), 1.93 (dd, J = 12.6, 7.1 Hz, 2H), 1.49 (dd, J = 15.0, 7.2 Hz, 2H), 1.27 (dd, J = 14.7, 7.2 Hz, 2H), 0.91 (d, J = 6.7 Hz, 6H). HRMS (ESI): m/z, calcd for C28H38N2O4 [M + H]⁺, 467.2904; found: 467.2895.

### Example 16. Synthesis of (E)-2-methoxy-4-(8-methylnonanamide)phenyl 2-([[1,4'-bipiperidin]-1'-yl) propanoate hydrochloride (compound A-16)

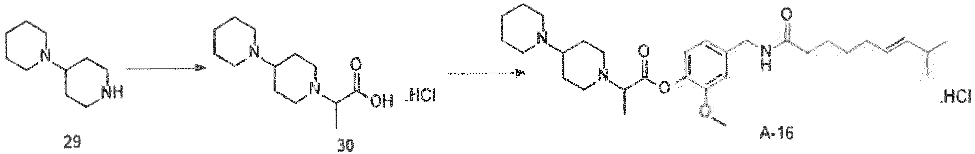

Reaction reagents and conditions: (a) 2-chloropropionic acid, EA, rt, 3 h, 80.3%; (b) *trans*-8-methyl-*N*-vanillyl-6-nonenamide, EDCI, DMAP, sodium chloride, concentrated sulfuric acid, DCM, EA, rt, 3 h, 63.2%.

### Synthesis of 2-([1,4'-bipiperidin]-1'-yl)propionic acid hydrochloride (compound 30)

Compound 29 (2 g, 11.89 mmol) was dissolved in a certain amount of dichloromethane. The reaction system was cooled to 5 °C in an ice bath, slowly added with 2-chloropropionic acid (0.65 g, 5.95 mmol), and reacted at room temperature for 3 h. Then the reaction system was concentrated under reduced pressure to remove dichloromethane, and the residue was added with a certain amount of ethyl acetate, and filtered in vacuum to remove the precipitated salts. The filtrate was added with hydrochloric acid to adjust the pH to 3-4, concentrated by rotary evaporation to remove the solvent, and re-crystallized from ethanol to give a white solid (compound 30).

### Synthesis of (E)-2-methoxy-4-(8-methylnonanamide)phenyl 2-([[1,4'-bispiperidin]-1'-yl)propionate hydrochloride (compound A-16)

Compound 30 (0.22 g, 0.79 mmol) was dissolved in a certain amount of dichloromethane. The reaction system was cooled to 5 °C in an ice bath, slowly added with *trans*-8-methyl-*N*-vanillyl-6-nonenamide (0.2 g, 0.65 mmol), 1-(3-dimethylaminopropyl)-3-ethylcarbodiimide hydrochloride (0.19 g, 0.98 mmol) and 4-dimethylaminopyridine (0.016 g, 0.13 mmol), and stirred at room temperature for 2 h. Then the reaction system was concentrated under reduced to remove the solvent, and the residue was added with a certain amount of ethyl acetate, washed with hydrochloric acid (2 N) three times, washed with water twice, dried over anhydrous sodium sulfate, and filtered. The filtrate was concentrated to give an oil. The oil was dissolved in a certain amount of ethyl acetate. The reaction system was purged with HCl gas generated by the reaction of sodium chloride with concentrated sulfuric acid until the pH was adjusted to 3-4, stirred at room temperature for 1 h, and filtered in vacuum to give a white solid (compound A-16). 1H NMR (300 MHz, MeOD) δ 7.16 - 7.05 (m, 2H), 6.91 (d, J = 8.1 Hz, 1H), 5.42-5.29 (m, 2H), 4.63 (d, J = 6.9 Hz, 2H), 4.36 (s, 2H), 3.83 (s, 3H), 3.57 (dd, J = 27.6, 15.3 Hz, 5H), 3.16-3.00 (m, 3H), 2.52 (d, J = 12.2 Hz, 2H), 2.22 (dt, J = 12.2, 6.4 Hz, 5H), 1.96 (t, J = 11.3 Hz, 5H), 1.83 (t, J = 9.0 Hz, 4H), 1.62 (dt, J = 15.3, 7.6 Hz, 3H), 1.36 (dt, J = 14.9, 7.6 Hz, 3H), 0.94 (d, J = 6.7 Hz, 6H). HRMS (ESI): m/z, calcd for C31H49N3O4 [M + H]⁺, 528.3796; found: 528.3796.

### Example 17. Synthesis of (E)-2-methoxy-4-((8-methylnon-6-enamido)methyl)phenyldiethylglycine oxalate (compound A-17)

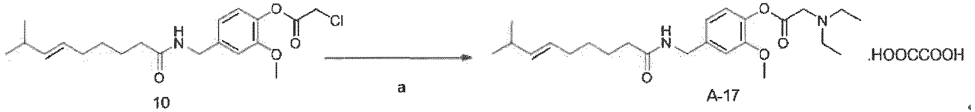

### Reaction reagents and conditions: (a) diethylamine, ethyl acetate, oxalic acid, 60 °C, 3 h, 62.5%. Synthesis of (E)-2-methoxy-4-((8-methylnon-6-enamido)methyl)phenyldiethylglycine oxalate (compound A-17)

Compound 10 (4.65 g, 12.31 mmol) and diethylamine (3.56 g, 48.67 mmol) were dissolved in a certain amount of ethyl acetate. The reaction system was heated to 60 °C and reacted for 3 h. Then the reaction system was added with a certain amount of water followed by liquid separation. The organic phase was added with dilute hydrochloric acid to adjust the pH to 1-2, and the aqueous phase was added with a certain amount of ethyl acetate and adjusted to pH 8-9 followed by liquid separation. The organic phase was concentrated to give an oil. The oil was dissolved in a certain amount of ethyl acetate, added with oxalic acid dihydrate (1.16 g, 9.10 mmol), and reacted at 40 °C for 15 min. Then the reaction system was cooled to room temperature, stirred for 1 h, and filtered in vacuum to give an off-white solid (3.62 g, 62.5%). ¹H NMR (300 MHz, DMSO-d₆): 8.35 (t, J = 5.9 Hz, 1H), 7.06 (dd, J = 16.3, 4.9 Hz, 2H), 6.84 (dd, J = 8.2, 1.7 Hz, 1H), 5.36-5.32 (m, 2H), 4.26 (d, J = 3.9 Hz, 2H), 3.98 (s, 2H), 3.75 (s, 3H), 2.95 (dd, J = 7.0, 3.9 Hz, 4H), 2.24-2.10 (m, 3H), 1.94 (dd, J = 12.9, 6.9 Hz, 2H), 1.57-1.45 (m, 2H), 1.34-1.28 (m, 2H),1.14 (t, J = 7.2 Hz, 6H), 0.92 (d, J = 6.7 Hz, 6H). MS (ESI): m/z, [M + H]⁺, 419.45.

### Example 18. Synthesis of (E)-2-methoxy-4-((8-methylnon-6-enamido)methyl)phenyldimethylglycine oxalate (compound A-18)

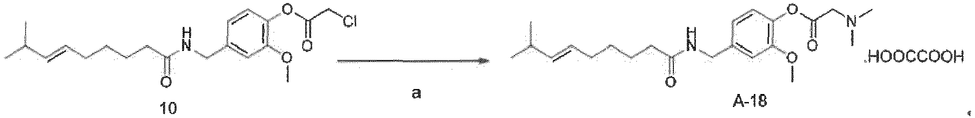

Reaction reagents and conditions: (a) dimethylamine hydrochloride, triethylamine, ethyl acetate, oxalic acid, 60 °C, 3 h, 23.5%.

### Synthesis of (E)-2-methoxy-4-((8-methylnon-6-enamido)methyl)phenyldimethylglycine oxalate (compound A-18)

Compound 10 (21 g, 54.99 mmol), dimethylamine hydrochloride (27.9 g, 342 mmol) and triethylamine (33.3 g, 333 mmol) were dissolved in a certain amount of ethyl acetate. The reaction system was heated to 60 °C and reacted for 4 h. Then the reaction system was added with a certain amount of water followed by liquid separation. The organic phase was added with dilute hydrochloric acid to adjust the pH to 1-2, and the aqueous phase was added with a certain amount of ethyl acetate and adjusted to pH 8-9 followed by liquid separation. The organic phase was concentrated to give an oil. The oil was dissolved in a certain amount of ethyl acetate, added with oxalic acid dihydrate (1.60 g, 12.70 mmol), and reacted at 40 °C for 15 min. Then the reaction system was cooled to room temperature, stirred for 1 h, and filtered in vacuum to give an off-white solid (6.2 g, 23.5%). ¹H NMR (300 MHz, DMSO-d₆): 8.35 (t, J = 5.9 Hz, 1H), 7.07 (dd, J = 16.3, 4.9 Hz, 2H), 6.85 (dd, J = 8.2, 1.7 Hz, 1H), 5.40-5.28(m, 2H), 4.26 (d, J = 3.9 Hz, 2H), 3.93 (s, 2H), 3.75 (s, 3H), 2.59 (s, 6H), 2.22-2.12 (m, 3H), 1.97 (dd, J = 12.9, 6.9 Hz, 2H), 1.57-1.45 (m, 2H), 1.34-1.27 (m, 2H), 0.92 (d, J = 6.7 Hz, 6H).MS (ESI): m/z, [M + H]⁺, 391.25.

### Example 19. Determination of solubility of compounds disclosed herein

To determine the solubility of the compounds disclosed herein in water, 10 mg of each of the compounds according to the examples of the present invention was added to a colorimetric tube, followed by the addition of 0.1 mL of water. The reaction system was sonicated for 5 min, and if the compound was not dissolved, 0.1 mL of water was supplemented until no undissolved material was observed. After the solubility measurement, it was found that the solubility of the compounds according to the examples of the present invention in water was much greater than that of the parent drug *trans*-8-methyl-*N*-vanillyl-6-nonenamide (solubility: 0.00841 mg/mL). The results are shown in Table 19-1.

**Table 19-1. Solubility of compounds disclosed herein**

| Examples | Solubility |
|---|---|
| Compound A-1 | 244mg/mL |
| Compound A-14 | 164mg/mL |
| Compound A-15 | 105mg/mL |
| Compound A-16 | 101mg/mL |
| Compound A-17 | 175mg/mL |
| Compound A-18 | 70mg/mL |
| *Trans*-8-methyl-*N*-vanillyl-6-nonenamide | 0.00841mg/mL |

### Example 20. In vitro pH stability of compounds disclosed herein

The compounds according to the examples of the present invention were incubated in the different buffers specified. The incubation was performed at room temperature, and samples were collected at specific time points. The formation of *trans*-8-methyl-*N-*vanillyl-6-nonenamide and the consumption of the starting compound were analyzed by HPLC, and the release half-life was calculated. The experiment demonstrated that the compounds disclosed herein have a certain stability at pH levels suitable for the preparation of pharmaceutical preparations, and can be stored, processed under appropriate conditions and prepared as medicaments. The results are shown in Table 20-1.

**Table 20-1. Release half-life of compounds disclosed herein at different pH**

| Medium | Release half-life t_{1/2} | | | |
|---|---|---|---|---|
| | A-1 | A-16 | A-17 | A-18 |
| 0.1mol HCl | 68.6 h | About 82 h | About 104 h | About 59 h |
| pH4.5 | About 24.7 h | About 73 h | About 48 h | About 31 h |
| pH7.4 | About 45.7 min | About 157 h | About 1 h | About 3 h |

### Example 21. In vitro simulation of in vivo drug release

0.1 mg of each of the compounds according to the examples of the present invention was incubated with the plasma sample at physiological temperature. Samples were collected at different time points. 100 µL of drug-containing plasma was precisely measured out, added with 1 mL of methanol to precipitate protein, and centrifuged. The supernatant was collected, and injected for analysis. The percentage of *trans*-8-methyl-*N*-vanillyl-6-nonenamide released by the compound disclosed herein was measured, and the release half-life t_{1/2} was calculated. The experiments demonstrated that about 50% of the compound disclosed herein was converted to the parent drug in a relatively short time. The test results are shown in Table 21-1.

**Table 21-1. Release half-life of compounds disclosed herein**

| Compound | Release half-life t_{1/2} |
|---|---|
| Compound A-1 | < 1 min |
| Compound A-16 | 2.5 h |
| Compound A-17 | <1 min |
| Compound A-18 | <1 min |

### Example 22. Irritation test (1)

The equal amount of the compounds according to the examples of the present invention were compared with capsaicine in odor. The results showed that the compounds according to the examples of the present invention were all white and tasteless, and had significantly less irritation.

### Example 23. Irritation test (2)

The experiment was performed to demonstrate that the irritation of the compounds disclosed herein to the nasal mucosa and perinasal skin of the rats is less than that of *trans*-8-methyl-*N*-vanillyl-6-nonenamide*.*

### 23.1 Test drugs

Sample: compound A-17 solution (16.67 mg/mL, equimolar with *trans*-8-methyl-*N*-vanillyl-6-nonenamide). Positive control: *trans*-8-methyl-*N*-vanillyl-6-nonenamide solution (10 mg/mL). Negative control solution: normal saline and blank solvent.

### 23.2 Laboratory animals

12 SPF male SD rats weighed 180-220 g, room temperature of 22±2 °C, humidity of about 50%, ammonia concentration below 20 ppm, and regular ventilation. All rats were fed with complete nutritional feed and housed in IVC cage boxes, 4 rats in each boxes, with free access to food and water.

### 23.3 Test methods

### 23.3.1 Grouping

Healthy SD rats were randomly divided into 4 groups, i.e., a treatment group (i.e., compound A-17 group), a positive control group, negative control groups (i.e., normal saline group and blank solvent group); 4 rats in each of the treatment group and the positive control group, and 2 rats in each of the negative control groups.

### 23.3.2 Administration

During the test, 10 µL of the sample solution or control solution was quantitatively pipetted and carefully dropped into the left nostril of the rats; the rats were observed for 30 min immediately after the administration and behavioral changes were recorded.

### 23.3.3 Observation index

For general states: the eye closure and the hypokinesia symptoms, and the beginning and ending time and the severity of the symptoms of the rats within 30 min after the administration;
For local irritative symptoms: the symptoms such as the congestion and redness and swelling of the nasal cavity and periphery and nasal secretion, and the times of sneezing and nasal scratching symptoms of the rats within 10 min after the administration and within 10-30 min after the administration.

**Table 23-1. Scoring standard of congestion and redness and swelling of nasal cavity and periphery of rats**

| Irritative symptoms | Score |
|---|---|
| Normal | 0 |
| Mild hyperemia | 1 |
| Obvious congestion and redness and swelling | 2 |
| Severe redness and swelling with secretion | 3 |

The calculation method for the times of nasal scratching: according to continuous nasal scratching of the rats after irritation to nasal mucosa; a continuous nasal scratching of no more than 10 s is counted as once; a continuous scratching of more than 10 s is counted as twice or more times, (for example, a continuous scratching of 30 s is counted as 3 times).

### 23.4 Results

Data were processed using the software SPSS19 for non-parametric tests, P < 0.05 showed a statistically significant difference.

### 23.4.1 General state observation

Negative control group: no eye closure or hypokinesia symptom was observed in the rats after the nasal administration of normal saline or blank solvent.

Positive control group: after the rats were administered *trans*-8-methyl-*N-*vanillyl-6-nonenamide*,* eye closure symptom that lasts for about 1.4 min was observed in 1 rat about 2 min later after the administration, and no eye closure symptom was observed in the other rats. Hypokinesia symptom that lasts for 3-12 min was observed in 3 of the 4 rats about 2 min later after the administration, with prostration and deep-breathing symptoms observed in 1 of the 3 rats.

Treatment group: no eye closure or hypokinesia was observed in the rats after the administration of compound A-17 during the observation.

### 23.4.2 Local irritation symptom observation

Negative control group: only transient and accidental sneezing or nasal scratching symptoms (less than 3 times) were observed in the rats after the nasal administration of normal saline or blank solvent, and the other symptoms such as the congestion and redness and swelling of nasal cavity and periphery were not observed.

Comparison of the local irritation of the compounds disclosed herein and *trans*-8-methyl-*N*-vanillyl-6-nonenamide to the nasal cavity of the rats: the severity of the congestion and redness and swelling of the nasal cavity and periphery of the rats induced by the treatment group (compound A-17) was obviously lower than that induced by the positive control group (*trans*-8-methyl-*N*-vanillyl-6-nonenamide administration group) (P < 0.001). The times of sneezing of the rats within 10 min and 10-30 min after the administration of the treatment group was less than that of the positive group (*trans*-8-methyl-*N*-vanillyl-6-nonenamide) (P < 0.001), the times of nasal scratching of the rats within 10 min after the administration of the treatment group was more than that of the positive control group (*trans*-8-methyl-*N*-vanillyl-6-nonenamide) (P < 0.001), but the times of nasal scratching of the rats within 10-30 min after the administration of the treatment group was less than that of the positive group (*trans*-8-methyl-*N*-vanillyl-6-nonenamide) (P < 0.001). The results are shown in Table 23-2.

**Table 23-2. Local irritation symptom statistics (n = 4, X±SD)**

| Irritation symptoms | | Positive control group (*trans*-8-methyl-*N*-vanillyl-6-nonenamide) | Treatment group (Compound A-17) | P value |
|---|---|---|---|---|
| Nasal cavity and nasal periphery | Redness and swelling score | 2.25±0.50 | 0.75±0.50 | <0.001 |
| Sneezing | Times within 0-30 min | 8.75±4.65 | 7.50±4.20 | <0.001 |
| | Times within 10-30 min | 7.25±9.88 | 1.75±0.96 | <0.001 |
| Nasal scratching | Times within 0-10 min | 6.50±4.36 | 13.50±3.87 | <0.001 |
| | Times within 10-30 min | 19.25±9.88 | 12.50±7.19 | <0.001 |

### Example 24. Study of analgesic effect on rats as models of pain after incision

### 24.1 Test materials and laboratory animals

Gas anesthesia machine for small animals, Shanghai Yuyan Instruments Co., Ltd., model number: ABS
Plantar tester, IITC Life Science, USA, model number: Series 8 Model 390
Von-Frey filaments, North Coast Medical Inc., model number: NC12775-99
Sterile syringe (1 mL), Shanghai Kindly Enterprise Development Group Co., Ltd.

### Laboratory animals

Species: SD rat Sex: male
Week age: 5-6 weeks body weight: 180-200 g
Quantity: 36

### 24.2 Preparation of rats as models of pain after incision

After 3-5 days of adaptive feeding, all rats were anesthetized with 3.5%-4% isoflurane/oxygen, and then were maintained with 1.5%-2% isoflurane. The drug (150 µL) was infiltrated by intraplantar injection shortly before the incision (1-2 min). Under sterile conditions, each rat was made a 1 cm longitudinal incision forward with a No.11 scalpel blade at 0.5 cm posterior to the metatarsals, the fascia and muscle were separated, the flexor was raised, and a longitudinal incision was made via blunt dissection, leaving the muscle source and insertion intact. After the hemostasis by light pressure, the drug was injected into the flexor (30 µL) before the wound was closed, and the skin was closed with two interrupted stitches at the incision site with a No. 5 nylon thread.

### 24.3 Grouping and administration

The modeled rats were randomly divided into 6 groups of 6 animals each: the high-dose (HD), medium-dose (MD) and low-dose (LD) groups of compound A-17, the LD and HD groups of *trans*-8-methyl-*N*-vanillyl-6-nonenamide, and a blank preparation group. Mode of administration: sample or control (150 µL) was infiltrated by intraplantar injection 1 min before the incision, and injected into the flexor (30 µL) before the wound was stitched. The specific grouping and administration design are shown in Table 24-1.

**Table 24-1. Administration design**

| **Grouping** | **Administration concentration (mg/mL)** | **Administration volume (mL/animal)** | **Administration dose (mg/animal)** | **Number of Animals*** | **Mode of administration** |
|---|---|---|---|---|---|
| A-17 (LD) | 0.83 | 0.18 | 0.15 | 6 | 150 µL of the compound was injected subcutaneously in foot 1-2 min before the incision, and 30 µE of the compound was injected in the flexor before the incision suture |
| A-17 (MD) | 1.665 | 0.18 | 0.3 | 6 | |
| A-17 (HD) | 3.33 | 0.18 | 0.6 | 6 | |
| *Trans-*8-methyl*-N-*vanillyl-6-nonenamide (LD) | 0.5 | 0.18 | 0.09 | 6 | |
| *Trans-*8*-*methyl*-N-*vanillyl-6-nonenamide (HD) | 2 | 0.18 | 0.36 | 6 | |
| Blank preparation | / | 0.18 | / | 6 | |

| | | | | | |
|---|---|---|---|---|---|
| ^{∗}: each animal received two tests after the administration, i.e., mechanical allodynia test and thermal hyperalgesia test. | | | | | |

### 24.4 Test results

### Mechanical allodynia test in rats - Von Frey method

The mechanical paw withdrawal threshold (PWT) of rats was tested as the pain threshold by irritating the middle plantar of hind limbs with Von Frey monofilaments at different time points after the administration. The Von Frey filaments were in 7 strengths, and the irritation intensity was increased approximately logarithmically (equivalent to 2 g, 4 g, 6 g, 8 g, 10 g, 15 g, 26 g). The rats was placed in a customized plexiglass box with a metal grid laid on the bottom, and acclimatized for at least 15 min before the test. The plantar surface of the hind paw of rats was contacted with the filaments, and certain pressure was applied to slightly bend the filaments. The filament strength was gradually increased with each filament tested 3 times on each plantar surface, and the pain threshold was defined as the lowest strength at which at least two paw withdrawals occurred over 3 consecutive tests. The test was performed at 1 h, 4 h, 24 h, 48 h, 72 h, 96 h and 168 h after the administration.

### Thermal hyperalgesia test in rats - Hargreaves method

The thermal withdrawal latency (TWL) of rats was measured using a IITC 390 thermal stimulation pain tester (Life science, USA). A 15 cm × 15 cm × 15 cm transparent plexiglass box was placed on the glass plate of the instrument, and the rats in the box were acclimatized for 10 min to rest before the test. When using the thermal radiation stimulator, the guide light source from the test head below the paw was first moved to the bottom of the paw of the rats, with the position determined by the reflector, and the heating light beam was then started to irradiate the bottom of the paw of the rats. The time from the start of irradiation to the occurrence of paw withdrawal in the rats was TWL. The light source irritation intensity was constant (90 W), and the automatic cut-off time was set to be 15 s to prevent the tissue from burning. The left and right feet of the rats were each tested for 5 times, with each interval of no less than 20 s. The maximum value and the minimum value of the 5 tests were discarded, only the average of the 3 middle values was taken as the test result of a certain specific time point, and then the average was taken. The average normal latency was about 8-10 s, and the rat whose measurements was less than 6 s or more than 12 s was considered to have a disqualified basal threshold, and cannot be given administration and subsequent tests. The test was performed at 1 h, 4 h, 24 h, 48 h, 72 h, 96 h and 168 h after the administration.

The test results were tested for Two-Way ANOVA using the statistical software Graphpad Prism 6, and P < 0.05 was considered a significant difference.

### Results

(1) Effect of A-17 on thermal hyperalgesia in rats as models of pain after incision.

The effect of A-17-HD, -MD and -LD administered by subcutaneous injection at the incision site on the thermal hyperalgesia in the rats as models of pain after incision was evaluated, with the blank preparation (Vehicle) as a negative control and *trans*-8-methyl-*N*-vanillyl-6-nonenamide-HD and -LD as positive controls. The results showed that A-17 relieved the thermal hyperalgesia in rats as models of pain after incision in a dose-dependent manner and a time-dependent manner, as shown in FIG. 2. According to statistical analysis, the A-17-HD group (n = 6) significantly increased the thermal withdrawal latency (TWL) value of the rats at 1-96 h after the surgery compared with the blank preparation group (n = 6), showing an relieving effect on the thermal hyperalgesia, namely, having an analgesic effect (P < 0.05). This suggests that its relieving effect on the thermal hyperalgesia effectively lasts up to 4 days. The A-17-MD group (n = 6) increased the TWL value of the rats at 1-72 h after the surgery, showing a better analgesic effect (P < 0.05). This suggests that its analgesic effect on the thermal hyperalgesia lasts for 3 days. The A-17-LD group (n = 6) increased the TWL value of rats at 1-24 h after the surgery. This suggests that its analgesic effect on the thermal hyperalgesia lasts for 1 day (P < 0.05). A dose-to-dose comparison of the A-17 preparations showed that the analgesic effect of the A-17-HD group was obviously better than that of the A-17-MD group (P < 0.05) at 1 h after the surgery, and was obviously better than that of the A-17-LD group (P < 0.05) at 1-96 h after the surgery. This suggests that the A-17-HD group shows an obvious dose dependence. In the positive controls, *trans*-8-methyl-*N*-vanillyl-6-nonenamide-HD group (n = 6) increased the TWL value of rats at 1-72 h after the surgery, showing a strong and lasting analgesic effect on the thermal hyperalgesia (for 3 days) (P < 0.05). The *trans*-8-methyl-*N*-vanillyl-6-nonenamide-LD group (n = 5) increased the TWL value of rats as models of pain after incision at 1 h after the surgery (P < 0.05), and had no significant effect on the TWL value thereafter (P > 0.05). The analgesic effect of the A-17-HD group (4 days) lasts longer than that of the *trans*-8-methyl-*N*-vanillyl-6-nonenamide-HD group (3 days), and the absolute TWL value of the A-17-HD group is slightly higher than that of the *trans*-8-methyl-*N*-vanillyl-6-nonenamide-HD group, but the difference was statistically insignificant (P > 0.05).

The area under the time-TWL value curve (AUC) of each group within 1-168 h after the surgery in FIG. 1 was calculated using the linear trapezoidal rule to indicate the overall thermal hyperalgesia of rats within 7 days (1-196 h) after the surgery. The overall analgesic effect of the drugs within 7 days was observed, and two-by-two comparisons were performed among the groups, as shown in FIG. 2. The results showed that the A-17-HD group and the A-17-MD group can effectively relieve the overall thermal hyperalgesia in the rats within 7 days after the incision (P < 0.001), while the A-17-LD group had no significant effect (P > 0.05). The analgesic effect of the A-17-HD group was obviously stronger than that of the A-17-LD group (P < 0.001). The *trans*-8-methyl-*N*-vanillyl-6-nonenamide-HD group was effective in relieving the overall thermal hyperalgesia (P < 0.01) in the rats within 7 days after the surgery, and the *trans*-8-methyl-*N*-vanillyl-6-nonenamide-LD group had no significant effect. The A-17-HD group was found to have significantly greater analgesic effect than the *trans*-8-methyl-*N*-vanillyl-6-nonenamide-HD group (P < 0.01).

(2) Effect of A-17 on mechanical allodynia in rats as models of pain after incision.

The relieving effect of A-17-HD, -MD and -LD administered by subcutaneous injection at the incision site on the mechanical allodynia in the rats as models of pain after incision was evaluated, with the blank preparation as a negative control and *trans*-8-methyl-*N*-vanillyl-6-nonenamide-HD and -LD as positive controls. The results are shown in FIG. 3. The A-17-HD group (n = 5) increased the PWT value of the rats at 1 h, 4 h and 48 h after the surgery compared with the blank preparation group (n = 5), showing an relieving effect on the mechanical allodynia, namely, having an analgesic effect (P < 0.05), after which its effect weakened until it disappeared. The A-17-MD group (n = 5) increased the PWT value of the rats at 24 h and 48 h after the surgery, showing a better analgesic effect (P < 0.05), after which it had a tendency to improve the PWT value but no statistical significance (P > 0.05). The A-17-LD group (n = 5) increased the PWT value of the rats at 1 h after the surgery (P < 0.05), after which its effect weakened until it disappeared. There was no significant difference among the A-17 dose groups (P > 0.05). The *trans*-8-methyl-*N*-vanillyl-6-nonenamide -HD group (n = 5) increased the PWT value of the rats at 4 h after the surgery, and had a tendency to improve the PWT value at the other time points but no statistical significance (P > 0.05). There was no significant effect on the PWT value of the rats as models of pain after incision in the *trans*-8-methyl-*N*-vanillyl-6-nonenamide-LD group (n = 5) at all time points after the surgery (P > 0.05). There was no significant difference between the A-17-HD group and the *trans*-8-methyl-*N*-vanillyl-6-nonenamide-HD group (P > 0.05).

The above test results suggest that: the A-17-HD group has a strong and lasting analgesic effect on thermal hyperalgesia induced by the incision pain of rats after the surgery, which can last for 4 days (1-96 h), and is superior to the *trans*-8-methyl-*N*-vanillyl-6-nonenamide-HD group (3 days), and the equal dose of *trans*-8-methyl-*N*-vanillyl-6-nonenamide; the A-17-MD group also has a strong analgesic effect, which lasts for 3 days (1-72 h); and the A-17-LD group exerts an analgesic effect only within 1 day (1-24 h). Furthermore, in terms of the intensity of the analgesic effect (TWL values at all time points and overall thermal hyperalgesia within 7 days), the analgesic effect of the A-17-HD group is significantly better than that of the A-17-MD group and that of the A-17-LD group. Therefore, A-17 relieve the thermal hyperalgesia induced by the incision pain after the surgery in a dose-dependent manner and a time-dependent manner, with the intensity and the duration of the analgesic effect better than those of the *trans*-8-methyl-*N*-vanillyl-6-nonenamide at the same molar dose.

The A-17-HD group has a certain analgesic effect on the mechanical allodynia induced by the incision pain after the surgery, which can last for 2 days (1-4 h, 48 h); the A-17-MD group also has a certain analgesic effect, which starts at 24 h after the surgery and lasts for 1 day (24-48 h); and the A-17-LD group exerts an analgesic effect only at 1 h after the surgery. There is no significant difference among the A-17 dose groups. The *trans*-8-methyl-*N*-vanillyl-6-nonenamide-HD group has a certain analgesic effect only at 4 h after the surgery; and the *trans*-8-methyl-*N*-vanillyl-6-nonenamide-LD group has no analgesic effect. There is no significant difference between the A-17-HD group and the *trans*-8-methyl-*N*-vanillyl-6-nonenamide-HD group.

### Example 25. Pharmacokinetics of compounds disclosed herein in vivo

### Example 25. Clinical compatibility stability

10 mg of each of the compounds according to the examples of the present invention was added with 10 mL of 0.9% sodium chloride and glucose injection. The reaction system was shaken to mix well, and the test tubes were left to stand at room temperature and sampled at 1 h, 2 h, 4 h, 6 h and 8 h. The appearance of the sample was observed, and the related substances and pH value were determined. It suggests that the compounds disclosed herein have no obvious pH change within 6 h and have an impurity change of less than 10% within 8 h, feature good compatibility stability and are suitable for clinical application. The clinical preparation data for compound A-1 are shown in the following table.

**Table 25-1. Clinical compatibility stability**

| 0.9% sodium chloride injection (25 °C) | | | | | | | |
|---|---|---|---|---|---|---|---|
| Investigation time (h) | | 0 | 1 | 2 | 4 | 6 | 8 |
| Character | | Clear liquid | | | | | |
| pH | | 3.39 | 3.38 | 3.37 | 3.34 | 3.31 | / |
| Related substances | Capsaicine | 2.57 | 3.22 | 3.84 | 4.95 | 6.1 | 7.22 |
| | Total impurities | 6.25 | 6.9 | 7.52 | 8.59 | 9.77 | 10.89 |
| Glucose injection (25 °C) | | | | | | | |
| Investigation time (h) | | 0 | 1 | 2 | 4 | 6 | 8 |
| Character | | Clear liquid | | | | | |
| pH | | 3.30 | 3.31 | 3.28 | 3.26 | 3.21 | / |
| Related substances | Capsaicine | 2.69 | 3.64 | 4.24 | 5.62 | 6.94 | 8.32 |
| | Total impurities | 6.46 | 7.41 | 8.00 | 9.38 | 10.7 | 12.02 |

The examples of the present invention have been described above. However, the present invention is not limited to the above examples. Any modification, equivalent, improvement and the like made without departing from the spirit and principle of the present invention shall fall within the protection scope of the present invention.

## Claims

1. A compound of formula I, or a stereoisomer, a tautomer, a solvate, a polymorph or a pharmaceutically acceptable salt thereof: 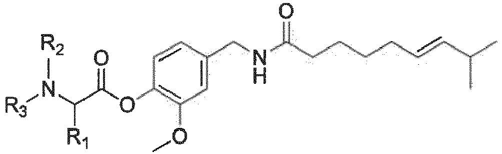 wherein,
R₁ is selected from H, halogen, hydroxyl, amino, and the following groups unsubstituted or optionally substituted with one, two or more R: a (C₁-C₁₂) aliphatic hydrocarbyl, a (C₁-C₁₂) aliphatic hydrocarbyl optionally comprising one, two or more heteroatoms, a C₃₋₁₂ cycloalkyl, a 3-12 membered heterocycloalkyl, a C₆₋₂₀ aryl, a 5-14 membered heteroaryl, S(=O)₂NH₂, -S(=O)₂NH-(C₁-C₁₂)aliphatic hydrocarbyl, and -S(=O)₂N((C₁-C₁₂)aliphatic hydrocarbyl)₂;
R₂ and R₃ are each independently selected from H, halogen, hydroxyl, amino, and the following groups unsubstituted or optionally substituted with one, two or more R: a (C₁-C₁₂)aliphatic hydrocarbyl, a (C₁-C₁₂)aliphatic hydrocarbyl optionally comprising one, two or more heteroatoms, a C₃₋₁₂ cycloalkyl, a 3-12 membered heterocycloalkyl, a C₆₋₂₀ aryl, a 5-14 membered heteroaryl, S(=O)₂NH₂,-S(=O)₂NH-(C₁-C₁₂)aliphatic hydrocarbyl, and -S(=O)₂N((C₁-C₁₂)aliphatic hydrocarbyl)₂, and at least one of R₂ and R₃ is not H; or R₂ and R₃, together with an N atom connected thereto, form an *N*-containing 3-12 membered heterocycloalkyl or an N-containing 5-14 membered heteroaryl unsubstituted or optionally substituted with one, two or more R;
the R is selected from halogen, CN, OH, NH₂, COOH, =O, a (C₁-C₁₂)aliphatic hydrocarbyl, a (C₁-C₁₂)aliphatic hydrocarbyl optionally comprising one, two or more heteroatoms, a C₃₋₁₂ cycloalkyl, a 3-12 membered heterocycloalkyl, a C₆₋₂₀ aryl, and a 5-14 membered heteroaryl.

2. The compound of formula I, or the stereoisomer, the tautomer, the solvate, the polymorph or the pharmaceutically acceptable salt thereof according to claim 1, wherein the *N*-containing 3-12 membered heterocycloalkyl or the *N*-containing 5-14 membered heteroaryl may be selected from an *N*-containing 5-6 membered heterocycloalkyl and an *N*-containing 5-6 membered heteroaryl; R₂ and R₃ are each independently selected from the following groups unsubstituted or optionally substituted with one, two or more R: a (C₁-C₁₂)aliphatic hydrocarbyl, and a (C₁-C₁₂)aliphatic hydrocarbyl optionally comprising one, two or more heteroatoms; the halogen is selected from F, Cl, Br and I; the (C₁-C₁₂)aliphatic hydrocarbyl is selected from a C₁-C₁₂ alkyl, a C₂-C₁₂ alkenyl and a C₂-C₁₂ alkynyl.

3. The compound of formula I, or the stereoisomer, the tautomer, the solvate, the polymorph or the pharmaceutically acceptable salt thereof according to claim 1 or 2, wherein
the "(C₁-C₁₂) aliphatic hydrocarbyl optionally comprising one, two or more heteroatoms" may be selected from a (C₁-C₆)aliphatic hydrocarbyloxy, a (C₁-C₆)aliphatic hydrocarbylthio, a (C₁-C₆)aliphatic hydrocarbyloxy(C₁-C₆)aliphatic hydrocarbyl, a (C₁-C₆)aliphatic hydrocarbylthio(C₁-C₆)aliphatic hydrocarbyl, an *N*-(C₁-C₃)aliphatic hydrocarbylamino(C₁-C₆)aliphatic hydrocarbyl, an *N*,*N-*di-(C₁-C₃)aliphatic hydrocarbylamino(C₁-C₆)aliphatic hydrocarbyl, a (C₁-C₆)aliphatic hydrocarbyl-NH-, and an N((C₁-C₆)aliphatic hydrocarbyl)₂; preferably, the (C₁-C₁₂)aliphatic hydrocarbyl substituted with one, two or more R and optionally containing one, two or more heteroatoms may be selected from a -(C₁-C₆)aliphatic hydrocarbyl OC(=O)NH₂, a -(C₁-C₆)aliphatic hydrocarbyl OC(=O)NH(C₁-C₆)aliphatic hydrocarbyl, a -(C₁-C₆)aliphatic hydrocarbyl OC(=O)N((C₁-C₆)aliphatic hydrocarbyl)₂, a -(C₁-C₆)aliphatic hydrocarbyl OC(=O)(C₁-C₆)aliphatic hydrocarbyl, and a -(C₁-C₆)aliphatic hydrocarbyl C(=O)O(C₁-C₆)aliphatic hydrocarbyl.

4. The compound of formula I, or the stereoisomer, the tautomer, the solvate, the polymorph or the pharmaceutically acceptable salt thereof according to any of claims 1 to 3, wherein
R₁ may be selected from methyl, ethyl, n-propyl, isopropyl, *n*-butyl, isobutyl, *tert-butyl, n*-pentyl, isopentyl, neopentyl, n-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, 1-hexynyl, phenyl, thienyl, furyl, pyrrolyl, pyridinyl, pyridazinyl, pyrimidinyl, pyrazinyl, and triazinyl;
R₂ and R₃ may be each independently selected from the following groups unsubstituted or optionally substituted with one, two or more R: methyl, ethyl, n-propyl, isopropyl, *n*-butyl, isobutyl, *tert*-butyl, *n*-pentyl, isopentyl, neopentyl, *n*-hexyl, ethenyl, 1-propenyl, 2-propenyl, 1-methylethenyl, 1-butenyl, 1-ethylethenyl, 1-methyl-2-propenyl, 2-butenyl, 3-butenyl, 2-methyl-1-propenyl, 2-methyl-2-propenyl, 1-pentenyl, 1-hexenyl, ethynyl, 1-propynyl, 2-propynyl, 1-butynyl, 1-methyl-2-propynyl, 3-butynyl, 1-pentynyl, and 1-hexynyl; or R₂ and R₃, together with an N atom connected thereto, form the following groups optionally substituted with one, two or more R: tetrahydropyrrolyl, piperidinyl, and 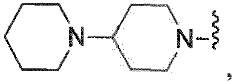 for example, may be selected from

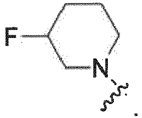

5. The compound of formula I, or the stereoisomer, the tautomer, the solvate, the polymorph or the pharmaceutically acceptable salt thereof according to any of claims 1 to 4, wherein the compound of formula I may be specifically selected from the following structures: 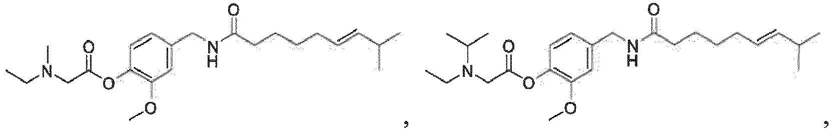 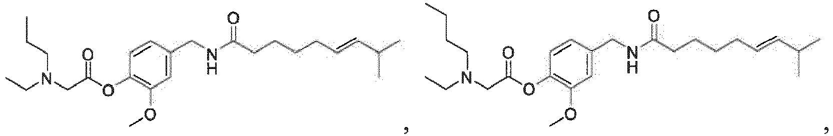 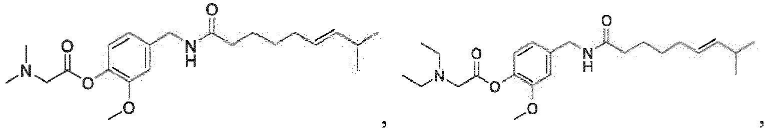 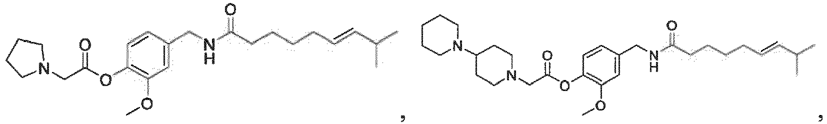 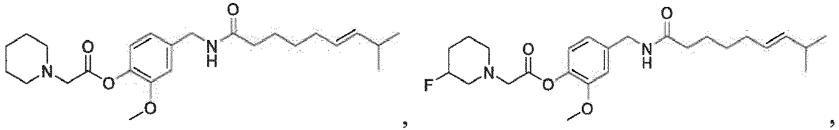 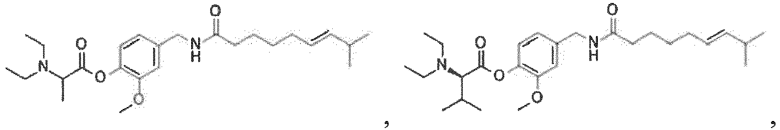 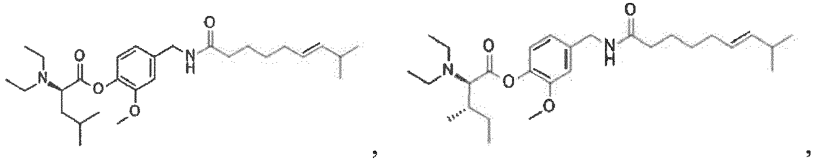 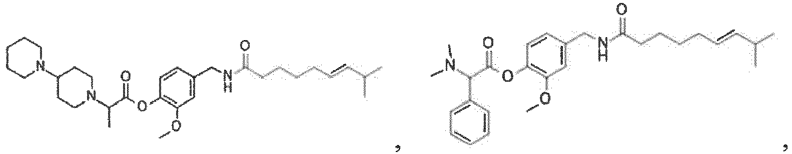 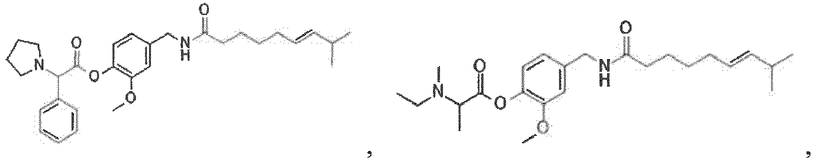 and

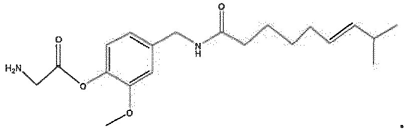

6. The compound of formula I, or the stereoisomer, the tautomer, the solvate, the polymorph or the pharmaceutically acceptable salt thereof according to any of claims 1 to 5, wherein the pharmaceutically acceptable salt of the compound of formula I may be selected from inorganic acid salts and organic acid salts, such as hydrochloride, oxalate, formate, and acetate.

7. The preparation method for the compound of formula I, or the stereoisomer, the tautomer, the solvate, the polymorph or the pharmaceutically acceptable salt thereof according to any of claims 1 to 6, comprising the following steps:
(1) reacting 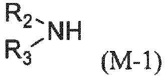 with chloroacetic acid to give 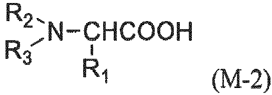 ; R₁, R₂ and R₃ are defined as those of the compound of formula I above;
(2) reacting the M-2 with *trans*-8-methyl-*N*-vanillyl-6-nonenamide to give the compound of formula I.

8. A pharmaceutical composition comprising the compound of formula I, or the stereoisomer, the tautomer, the solvate, the polymorph or the pharmaceutically acceptable salt thereof according to any of claims 1 to 6.

9. Use of the compound of formula I, or the stereoisomer, the tautomer, the solvate, the polymorph or the pharmaceutically acceptable salt thereof according to any of claims 1 to 6, or the pharmaceutical composition according to claim 8 in preparing a TRPV1 agonist.

10. The use according to claim 9, wherein the disease or condition is selected from post-operative pain, neuropathic pain, post-herpetic neuralgia, diabetic neuropathy, HIV-associated neuropathy, complex regional pain syndrome, cancer, nerve injury, cancer chemotherapy, vulvodynia, trauma, surgery, chronic musculoskeletal pain, lower back pain, osteoarthritis and rheumatoid arthritis-related conditions.
